# EUROPEAN PATENT SPECIFICATION

(11) **EP 4 419 011 B1**
(45) Date of publication and mention of the grant of the patent: **25.11.2026**
(21) Application number: 22806087.7
(22) Date of filing: 25.10.2022
(51) Int. Cl.: A61B 8/08, A61B 8/00

(54) **HIGH FIDELITY DOPPLER ULTRASOUND USING VESSEL DETECTION FOR RELATIVE ORIENTATION**
HIGH-FIDELITY-DOPPLER-ULTRASCHALL MIT GEFÄSSERKENNUNG FÜR RELATIVE AUSRICHTUNG
ECHOGRAPHIE DOPPLER HAUTE FIDELITE UTILISANT LA DETECTION DE VAISSEAUX POUR UNE ORIENTATION RELATIVE

(30) Priority: 25.10.2021 US 202163271621 P
(43) Date of publication of application: 28.08.2024
(73) Proprietor: Bard Access Systems, Inc., Salt Lake City, UT 84116 (US)
(72) Inventor: SOWARDS, Steffan, Salt Lake City, UT 84124 (US); MISENER, Anthony, K., Bountiful, UT 84010 (US); MCLAUGHLIN, William, Robert, Bountiful, UT 84010 (US)
(74) Representative: Hoffmann Eitle
(86) International application number: PCT/US2022/047727
(87) International publication number: WO 2023/076268

(56) References cited:
- WO-A1-2016/081023
- US-A1- 2019 239 848
- US-A1- 2020 359 990

## Description

### PRIORITY

This application claims the benefit of priority to U.S. Provisional Application No. 63/271,621, filed October 25, 2021.

### BACKGROUND

Ultrasound imaging is a widely accepted tool for guiding interventional instruments such as needles to targets such as blood vessels or organs in the human body. In order to successfully guide, for example, a needle to a blood vessel using ultrasound imaging, the needle is monitored in real-time both immediately before and after a percutaneous puncture in order to enable a clinician to determine the distance and the orientation of the needle to the blood vessel and ensure successful access thereto. However, through inadvertent movement of an ultrasound probe during the ultrasound imaging, the clinician can lose both the blood vessel and the needle, which can be difficult and time consuming to find again. In addition, it is often easier to monitor the distance and orientation of the needle immediately before the percutaneous puncture with a needle plane including the needle perpendicular to an image plane of the ultrasound probe. And it is often easier to monitor the distance and orientation of the needle immediately after the percutaneous puncture with the needle plane parallel to the image plane. As with inadvertently moving the ultrasound probe, the clinician can lose both the blood vessel and the needle when adjusting the image plane before and after the percutaneous puncture, which can be difficult and time consuming to find again. What is needed are ultrasound-imaging systems and methods thereof that can dynamically adjust the image plane to facilitate guiding interventional instruments to targets in at least the human body.

Doppler ultrasound is a noninvasive approach to estimating the blood flow through your blood vessels by bouncing high-frequency sound waves (ultrasound) off circulating red blood cells. A doppler ultrasound can estimate how fast blood flows by measuring the rate of change in its pitch (frequency). Doppler ultrasound may be performed as an alternative to more-invasive procedures, such as angiography, which involves injecting dye into the blood vessels so that they show up clearly on X-ray images. Doppler ultrasound may help diagnose many conditions, including blood clots, poorly functioning valves in your leg veins, which can cause blood or other fluids to pool in your legs (venous insufficiency), heart valve defects and congenital heart disease, a blocked artery (arterial occlusion), decreased blood circulation into your legs (peripheral artery disease), bulging arteries (aneurysms), and narrowing of an artery, such as in your neck (carotid artery stenosis). Doppler ultrasound may also detect a direction of blood flow within a blood vessel. US 2020/359990 A1 discloses an ultrasound device that is configured to indicate, via visual indicators on the ultrasound device, a direction to orient the ultrasound device for aligning the ultrasound device with the subjects anatomy, e.g. for aligning the device with a given blood vessel. The device may include an opening and, after the alignment, the clinician may accurately insert a needle or any medical device through the opening and into the blood vessel. US 2019/239848 A1 discloses an ultrasound imaging system for guiding a user to acquire ultrasound image data sets that can be analyzed for the presence of atherosclerosis. The processor is programmed to analyze ultrasound image data for a vessel of interest and to control a user interface that suggests how an operator should move a transducer to acquire ultrasound image data with the vessel of interest in a desired location. WO 2016/081023 A1 discloses an ultrasound imaging system that includes an interventional medical device having a first tracking element that generates tip location data based on an EM locator field. A processor circuit is configured to execute program instructions to generate an ultrasound image for display, and is configured to generate a positioning signal based on the tip location data and the probe location data to dynamically position the active ultrasound transducer array so that the two-dimensional ultrasound slice data includes the distal tip of the interventional medical device.

Disclosed herein are systems and methods for combining ultrasound imaging with doppler ultrasound to establish an orientation of the ultrasound image plane with respect to a blood vessel within the ultrasound image.

### SUMMARY

Disclosed herein is an ultrasound-imaging system according to claim 1. In some embodiments, the notification is tactile, audible, visual, or any combination thereof.

In some embodiments, the ultrasound image of a blood vessel defines an elliptical shape and in further embodiments, determining the misalignment includes: (i) identifying a length and a width of the elliptical shape, (ii) calculating a parameter relating to a difference between the length and the width, and (iii) comparing the calculated parameter with a parameter limit stored in memory.

In some embodiments, the ultrasound probe includes doppler ultrasound capability, and the operations further include determining a direction of blood flow within the blood vessel with respect the image plane based on doppler ultrasound data. In further embodiments, rendering the ultrasound image of the blood vessel includes superimposing an indicum atop the ultrasound image of the blood vessel, where the indicum indicates the direction of the blood flow.

The operations may further include: (i) comparing the ultrasound image of the blood vessel with one or more ultrasound images of blood vessels stored in memory, where the one or more ultrasound images pertain to a defined medical procedure, and (ii) providing a notification when, as a result of the comparison, an orientation of the image plane of the ultrasound image of the blood vessel is determined to be opposite to an orientation of a corresponding image plane of the one or more ultrasound images. In some embodiments, comparing the ultrasound image includes comparing the spatial positioning of the blood vessel in relation to adjacent anatomical elements in the ultrasound image of the blood vessel with the spatial positioning of a corresponding blood vessel in relation to corresponding adjacent anatomical elements in the one or more ultrasound images.

The ultrasound probe may, in some embodiments, further include an array of magnetic sensors configured to convert magnetic signals from a magnetized medical device into corresponding electrical signals of the magnetic signals for processing by the processor into distance and orientation information with respect to the blood vessel for display of an iconographic representation of the medical device on the display screen. In some embodiments, the distance and orientation of the activated ultrasonic transducers is adjusted with respect to the blood vessel so that when the medical device is brought into proximity of the ultrasound probe, a device image plane is established by the activated ultrasonic transducers that is perpendicular or parallel to a medical-device plane including the medical device for accessing the blood vessel with the medical device.

The system may further include: (1) a stand-alone optical interrogator communicatively coupled to the console or an integrated optical interrogator integrated into the console, where the optical interrogator is configured to (i) emit input optical signals, (ii) receive reflected optical signals, and (iii) convert the reflected optical signals into corresponding electrical signals of the optical signals for processing by the processor into distance and orientation information with respect to the blood vessel for rendering an iconographic representation of a medical device on the display; and (2) an optical-fiber stylet configured to convey the input optical signals from the optical interrogator to a number of fiber Bragg grating ("FBG") sensors along a length of the optical-fiber stylet and the reflected optical signals from the number of FBG sensors back to the optical interrogator, the optical-fiber stylet configured to be disposed in a lumen of the medical device.

In some embodiments, the system may further include an accelerometer, a gyroscope, a magnetometer, or a combination thereof configured to provide positional-tracking data to the console, and the processor is further configured to execute the instructions for processing the positional-tracking data to adjust of the distance of the activated ultrasonic transducers from the blood vessel, the orientation of the activated ultrasonic transducers to the blood vessel, or both the distance and the orientation of the activated ultrasonic transducers with respect to the blood vessel. In further embodiments, the distance and the orientation of the activated ultrasonic transducers is maintained with respect to the blood vessel when the ultrasound probe is inadvertently moved with respect to the blood vessel.

Also defined herein is a method according to claim 9. In some embodiments of the method, the notification is tactile, audible, visual, or any combination thereof.

In some embodiments of the method, the ultrasound image of a blood vessel defines an elliptical shape and in further embodiments, determining the misalignment includes identifying a length and a width of the elliptical shape, calculating a parameter relating to a difference between the length and the width, and comparing the calculated parameter with a parameter limit stored in memory.

In some embodiments of the method the ultrasound probe includes doppler ultrasound capability, and the method further includes determining a direction of blood flow within the blood vessel with respect the image plane based on doppler ultrasound data.

In some embodiments of the method, rendering the ultrasound image of the blood vessel includes superimposing an indicum atop the ultrasound image of the blood vessel, where the indicum indicates the direction of the blood flow.

The method may further include: (i) comparing the ultrasound image of the blood vessel with one or more ultrasound images of blood vessels stored in memory, the one or more ultrasound images pertaining to a defined medical procedure, and (ii) providing a notification when, as a result of the comparison, an orientation of the image plane of the ultrasound image of the blood vessel is determined to be opposite to the orientation of the image plane of the one or more ultrasound images. In some embodiments of the method, comparing the ultrasound image includes comparing the spatial positioning of the blood vessel in relation to adjacent anatomical elements in the ultrasound image of the blood vessel with the spatial positioning of a corresponding blood vessel in relation to corresponding adjacent anatomical elements in the one or more ultrasound images.

In some embodiments, the method further includes: (i) converting magnetic signals from a magnetized medical device with an array of magnetic sensors of the ultrasound probe into corresponding electrical signals of the magnetic signals, (ii) processing the corresponding electrical signals of the magnetic signals with the processor into distance and orientation information with respect to the blood vessel, and (iii) rendering an iconographic representation of the medical device on the display.

In some embodiments, the method further includes: (i) emitting input optical signals, receiving reflected optical signals, and converting the reflected optical signals into corresponding electrical signals of the optical signals by a stand-alone optical interrogator communicatively coupled to the console or an integrated optical interrogator integrated into the console, (ii) conveying the input optical signals from the optical interrogator to a number of fiber Bragg grating ("FBG") sensors along a length of an optical-fiber stylet and the reflected optical signals from the number of FBG sensors back to the optical interrogator with the optical-fiber stylet disposed in a lumen of the medical device, (iii) processing the corresponding electrical signals of the optical signals with the processor into distance and orientation information with respect to the blood vessel, and (iv) rendering an iconographic representation of a medical device on the display.

In some embodiments, the method further includes adjusting the distance and orientation of the activated ultrasonic transducers with respect to a blood vessel when the medical device is brought into proximity of the ultrasound probe, thereby establishing a device image plane by the activated ultrasonic transducers perpendicular or parallel to a medical-device plane including the medical device for accessing the blood vessel with the medical device.

In some embodiments, the method further includes: providing positional-tracking data to the console from an accelerometer, a gyroscope, a magnetometer, or a combination thereof of the ultrasound probe; and processing the positional-tracking data with the processor for the adjusting of the distance of the activated ultrasonic transducers from the blood vessel or area, the orientation of the activated ultrasonic transducers to the blood vessel, or both the distance and the orientation of the activated ultrasonic transducers with respect to the blood vessel.

In some embodiments, the method further includes maintaining the distance and the orientation of the activated ultrasonic transducers with respect to the blood vessel when the ultrasound probe is inadvertently moved with respect to the blood vessel.

These and other features of the concepts provided herein will become more apparent to those of skill in the art in view of the accompanying drawings and following description, which describe particular embodiments of such concepts in greater detail.

### DRAWINGS

FIG. 1 illustrates an ultrasound-imaging system and a patient in accordance with some embodiments.
FIG. 2 illustrates a block diagram of a console of the ultrasound-imaging system of FIG. 1 in accordance with some embodiments.
FIG. 3A illustrates an ultrasound probe of the ultrasound-imaging system imaging a blood vessel in accordance with some embodiments.
FIG. 3B illustrates an ultrasound image of the blood vessel of FIG. 3A on a display screen of the ultrasound-imaging system in accordance with some embodiments.
FIG. 4 illustrates the ultrasound probe of the ultrasound-imaging system configured as a 2-D ultrasound probe in accordance with some embodiments.
FIG. 5A illustrates activated ultrasonic transducers of an array of ultrasonic transducers of the ultrasound probe in accordance with some embodiments.
FIG. 5B illustrates the ultrasound image of the blood vessel of FIG. 3A obtained with the activated ultrasonic transducers of FIG. 5A on the display screen of the ultrasound-imaging system in accordance with some embodiments.
FIG. 6A illustrates the activated ultrasonic transducers of the ultrasound probe of FIG. 5A upon rotating the ultrasound probe without dynamic adjusting of the activated ultrasonic transducers in accordance with some embodiments.
FIG. 6B illustrates the ultrasound image of the blood vessel of FIG. 3A obtained with the activated ultrasonic transducers of FIG. 6A on the display screen of the ultrasound-imaging system in accordance with some embodiments.
FIG. 7A illustrates the activated ultrasonic transducers of the ultrasound probe of FIG. 5A upon rotating the ultrasound probe with dynamic adjusting of the activated ultrasonic transducers in accordance with some embodiments.
FIG. 7B illustrates the ultrasound image of the blood vessel of FIG. 3A obtained with the activated ultrasonic transducers of FIG. 7A on the display screen of the ultrasound-imaging system in accordance with some embodiments.
FIG. 8A illustrates the activated ultrasonic transducers of the array of ultrasonic transducers of the ultrasound probe in accordance with some embodiments.
FIG. 8B illustrates the ultrasound image of the blood vessel of FIG. 3A obtained with the activated ultrasonic transducers of FIG. 8A on the display screen of the ultrasound-imaging system in accordance with some embodiments.
FIG. 9A illustrates the activated ultrasonic transducers of the ultrasound probe of FIG. 8A upon translating the ultrasound probe without dynamic adjusting of the activated ultrasonic transducers in accordance with some embodiments.
FIG. 9B illustrates the ultrasound image of the blood vessel of FIG. 3A obtained with the activated ultrasonic transducers of FIG. 9A on the display screen of the ultrasound-imaging system in accordance with some embodiments.
FIG. 10A illustrates the activated ultrasonic transducers of the ultrasound probe of FIG. 10A upon translating the ultrasound probe with dynamic adjusting of the activated ultrasonic transducers in accordance with some embodiments.
FIG. 10B illustrates the ultrasound image of the blood vessel of FIG. 3A obtained with the activated ultrasonic transducers of FIG. 10A on the display screen of the ultrasound-imaging system in accordance with some embodiments.
FIG. 11 illustrates the activated ultrasonic transducers of the array of ultrasonic transducers of the ultrasound probe perpendicular to a medical-device plane of a magnetized medical device in accordance with some embodiments.
FIG. 12 illustrates the activated ultrasonic transducers of the array of ultrasonic transducers of the ultrasound probe perpendicular to the medical-device plane of the magnetized medical device after yawing the medical device and dynamically adjusting the activated ultrasonic transducers in accordance with some embodiments.
FIG. 13 illustrates the activated ultrasonic transducers of the array of ultrasonic transducers of the ultrasound probe perpendicular to the medical-device plane of the magnetized medical device after yawing the medical device and dynamically adjusting the activated ultrasonic transducers in accordance with some embodiments.
FIG. 14 illustrates the ultrasound probe of the ultrasound-imaging system configured as a linear ultrasound probe in accordance with some embodiments.
FIG. 15A illustrates activated ultrasonic transducers of an array of ultrasonic transducers of the ultrasound probe in accordance with some embodiments.
FIG. 15B illustrates the ultrasound image of the blood vessel of FIG. 3A obtained with the activated ultrasonic transducers of FIG. 15A on the display screen of the ultrasound-imaging system in accordance with some embodiments.
FIG. 16A illustrates the activated ultrasonic transducers of the ultrasound probe of FIG. 15A upon rotating the ultrasound probe without dynamic adjusting of the activated ultrasonic transducers in accordance with some embodiments.
FIG. 16B illustrates the ultrasound image of the blood vessel of FIG. 3A obtained with the activated ultrasonic transducers of FIG. 16A on the display screen of the ultrasound-imaging system in accordance with some embodiments.
FIG. 17A illustrates the activated ultrasonic transducers of the ultrasound probe of FIG. 15A upon rotating the ultrasound probe with dynamic adjusting of the activated ultrasonic transducers in accordance with some embodiments.
FIG. 17B illustrates the ultrasound image of the blood vessel of FIG. 3A obtained with the activated ultrasonic transducers of FIG. 17A on the display screen of the ultrasound-imaging system in accordance with some embodiments.
FIG. 18A illustrates the activated ultrasonic transducers of the array of ultrasonic transducers of the ultrasound probe in accordance with some embodiments.
FIG. 18B illustrates the ultrasound image of the blood vessel of FIG. 3A obtained with the activated ultrasonic transducers of FIG. 18A on the display screen of the ultrasound-imaging system in accordance with some embodiments.
FIG. 19A illustrates the activated ultrasonic transducers of the ultrasound probe of FIG. 18A upon translating the ultrasound probe without dynamic adjusting of the activated ultrasonic transducers in accordance with some embodiments.
FIG. 19B illustrates the ultrasound image of the blood vessel of FIG. 3A obtained with the activated ultrasonic transducers of FIG. 19A on the display screen of the ultrasound-imaging system in accordance with some embodiments.
FIG. 20A illustrates the activated ultrasonic transducers of the ultrasound probe of FIG. 20A upon translating the ultrasound probe with dynamic adjusting of the activated ultrasonic transducers in accordance with some embodiments.
FIG. 20B illustrates the ultrasound image of the blood vessel of FIG. 3A obtained with the activated ultrasonic transducers of FIG. 20A on the display screen of the ultrasound-imaging system in accordance with some embodiments.
FIG. 21 illustrates the activated ultrasonic transducers of the array of ultrasonic transducers of the ultrasound probe perpendicular to the medical-device plane of the magnetized medical device in accordance with some embodiments.
FIG. 22 illustrates the activated ultrasonic transducers of the array of ultrasonic transducers of the ultrasound probe perpendicular to the medical-device plane of the magnetized medical device after yawing the medical device and dynamically adjusting the activated ultrasonic transducers in accordance with some embodiments.
FIG. 23 illustrates the activated ultrasonic transducers of the array of ultrasonic transducers of the ultrasound probe perpendicular to the medical-device plane of the magnetized medical device after yawing the medical device and dynamically adjusting the activated ultrasonic transducers in accordance with some embodiments.
FIG. 24A illustrates a top view of the ultrasound probe of FIG. 1 placed on a patient where the ultrasound probe is rotated with respect to the blood vessel in accordance with some embodiments.
FIG. 24B illustrates an ultrasound image of the blood vessel resulting from the ultrasound probe placement of FIG. 24A in accordance with some embodiments.
FIG. 24C illustrates a side view of the ultrasound probe of FIG. 1 placed on a patient where the ultrasound probe is tilted with respect to the blood vessel in accordance with some embodiments.
FIG. 24D illustrates an ultrasound image of the blood vessel resulting from the ultrasound probe placement of FIG. 24C in accordance with some embodiments.
FIG. 25A illustrates a side view of the ultrasound probe of FIG. 1 placed on a patient where the ultrasound probe is disposed in a first orientation with respect to a vein and an artery in accordance with some embodiments.
FIG. 25B illustrates an ultrasound image of the vein and artery resulting from the ultrasound probe placement of FIG. 25A in accordance with some embodiments.
FIG. 25C illustrates a side view of the ultrasound probe of FIG. 1 placed on a patient where the ultrasound probe is disposed in a second orientation opposite the first orientation of FIG. 25A in accordance with some embodiments.
FIG. 25D illustrates an ultrasound image of the vein and artery resulting from the ultrasound probe placement of FIG. 25C in accordance with some embodiments.

### DESCRIPTION

Before some particular embodiments are disclosed in greater detail, it should be understood that the particular embodiments disclosed herein do not limit the scope of the appended claims. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the claims. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, ultrasound-imaging systems and methods thereof are needed that can dynamically adjust the image plane to facilitate guiding interventional instruments to targets in at least the human body. Disclosed herein are dynamically adjusting ultrasound-imaging systems and methods thereof.

### Ultrasound-imaging systems

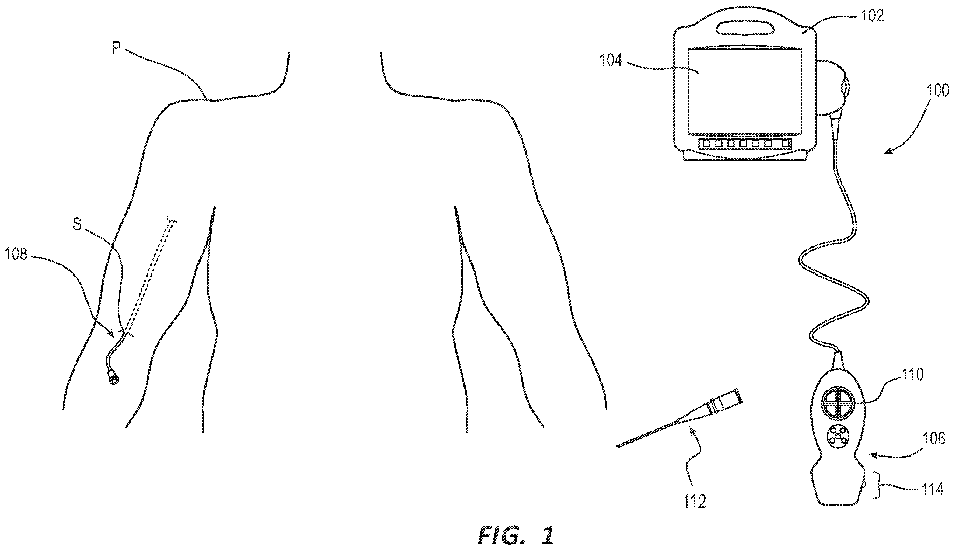

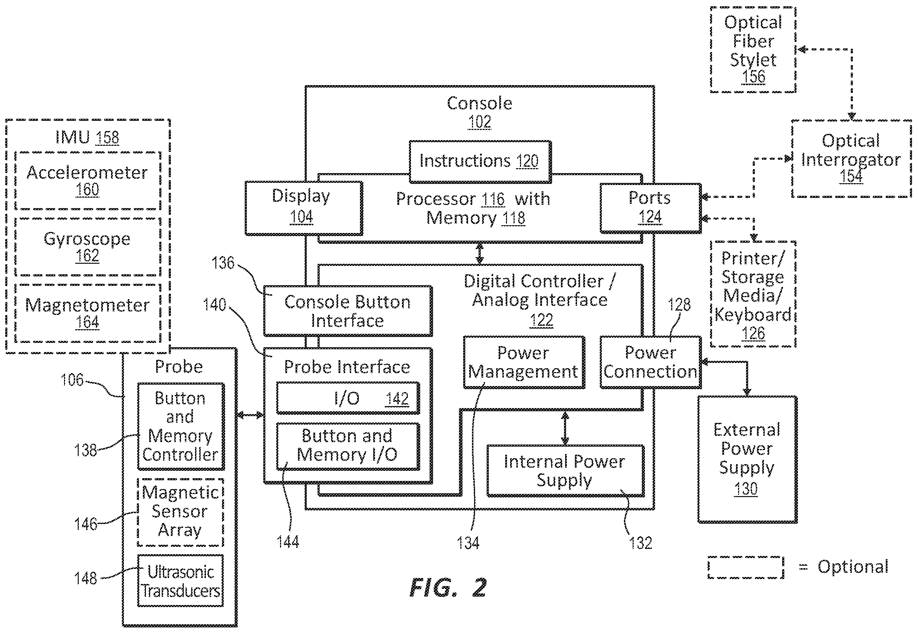

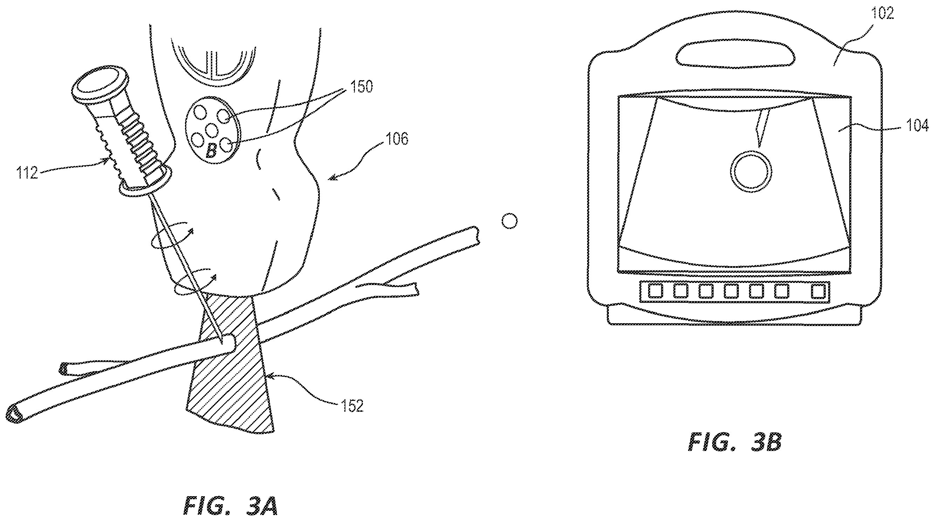

FIG. 1 illustrates an ultrasound-imaging system 100, a needle 112, and a patient *P* in accordance with some embodiments. FIG. 2 illustrates a block diagram of the ultrasound-imaging system 100 in accordance with some embodiments. FIG. 3A illustrates an ultrasound probe 106 of the ultrasound-imaging system 100 imaging a blood vessel of the patient P prior to accessing the blood vessel in accordance with some embodiments. FIG. 3B illustrates an ultrasound image of the blood vessel of FIG. 3A on a display screen 104 of the ultrasound-imaging system 100 with an iconographic representation of the needle 112 in accordance with some embodiments.

As shown, the ultrasound-imaging system 100 includes a console 102, the display screen 104, and the ultrasound probe 106. The ultrasound-imaging system 100 is useful for imaging a target such as a blood vessel or an organ within a body of the patient *P* prior to a percutaneous puncture with the needle 112 for inserting the needle 112 or another medical device into the target and accessing the target. Indeed, the ultrasound-imaging system 100 is shown in FIG. 1 in a general relationship to the patient P during an ultrasound-based medical procedure to place a catheter 108 into the vasculature of the patient P through a skin insertion site S created by a percutaneous puncture with the needle 112. It should be appreciated that the ultrasound-imaging system 100 can be useful in a variety of ultrasound-based medical procedures other than catheterization. For example, the percutaneous puncture with the needle 112 can be performed to biopsy tissue of an organ of the patient P.

The console 102 houses a variety of components of the ultrasound-imaging system 100, and it is appreciated the console 102 can take any of a variety of forms. A processor 116 and memory 118 such as random-access memory ("RAM") or non-volatile memory (e.g., electrically erasable programmable read-only memory ["EEPROM"]) is included in the console 102 for controlling functions of the ultrasound-imaging system 100, as well as executing various logic operations or algorithms during operation of the ultrasound-imaging system 100 in accordance with executable logic 120 therefor stored in the memory 118 for execution by the processor 116. For example, the console 102 is configured to instantiate by way of the logic 120 one or more processes for dynamically adjusting a distance of activated ultrasonic transducers 149 from a predefined target (e.g., blood vessel) or area, an orientation of the activated ultrasonic transducers 149 to the predefined target or area, or both the distance and the orientation of the activated ultrasonic transducers 149 with respect to the predefined target or area, as well as process electrical signals from the ultrasound probe 106 into ultrasound images. Dynamically adjusting the activated ultrasonic transducers 149 uses ultrasound-imaging data, magnetic-field data, shape-sensing data, or a combination thereof received by the console 102 for activating certain ultrasonic transducers of a 2-D array of the ultrasonic transducers 148 or moving those already activated in a linear array of the ultrasonic transducers 148. A digital controller/analog interface 122 is also included with the console 102 and is in communication with both the processor 116 and other system components to govern interfacing between the ultrasound probe 106 and other system components set forth herein.

The ultrasound-imaging system 100 further includes ports 124 for connection with additional components such as optional components 126 including a printer, storage media, keyboard, etc. The ports 124 can be universal serial bus ("USB") ports, though other types of ports can be used for this connection or any other connections shown or described herein. A power connection 128 is included with the console 102 to enable operable connection to an external power supply 130. An internal power supply 132 (e.g., a battery) can also be employed either with or exclusive of the external power supply 130. Power management circuitry 134 is included with the digital controller/analog interface 122 of the console 102 to regulate power use and distribution.

The display screen 104 is integrated into the console 102 to provide a GUI and display information for a clinician during such as one-or-more ultrasound images of the target or the patient P attained by the ultrasound probe 106. In addition, the ultrasound-imaging system 100 enables the distance and orientation of a magnetized medical device such as the needle 112 to be superimposed in real-time atop an ultrasound image of the target, thus enabling a clinician to accurately guide the magnetized medical device to the intended target. Notwithstanding the foregoing, the display screen 104 can alternatively be separate from the console 102 and communicatively coupled thereto. A console button interface 136 and control buttons 110 (*see* FIG. 1) included on the ultrasound probe 106 can be used to immediately call up a desired mode to the display screen 104 by the clinician for assistance in an ultrasound-based medical procedure. In some embodiments, the display screen 104 is an LCD device.

The ultrasound probe 106 is employed in connection with ultrasound-based visualization of a target such as a blood vessel (*see* FIG. 3A) in preparation for inserting the needle 112 or another medical device into the target. Such visualization gives real-time ultrasound guidance and assists in reducing complications typically associated with such insertion, including inadvertent arterial puncture, hematoma, pneumothorax, etc. As described in more detail below, the ultrasound probe 106 is configured to provide to the console 102 electrical signals corresponding to both the ultrasound-imaging data, the magnetic-field data, the shape-sensing data, or a combination thereof for the real-time ultrasound guidance.

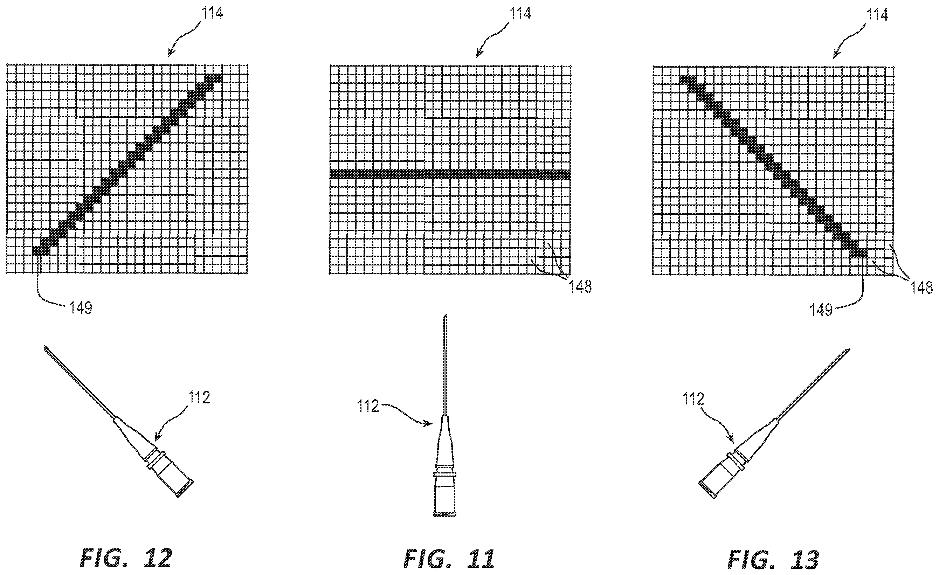

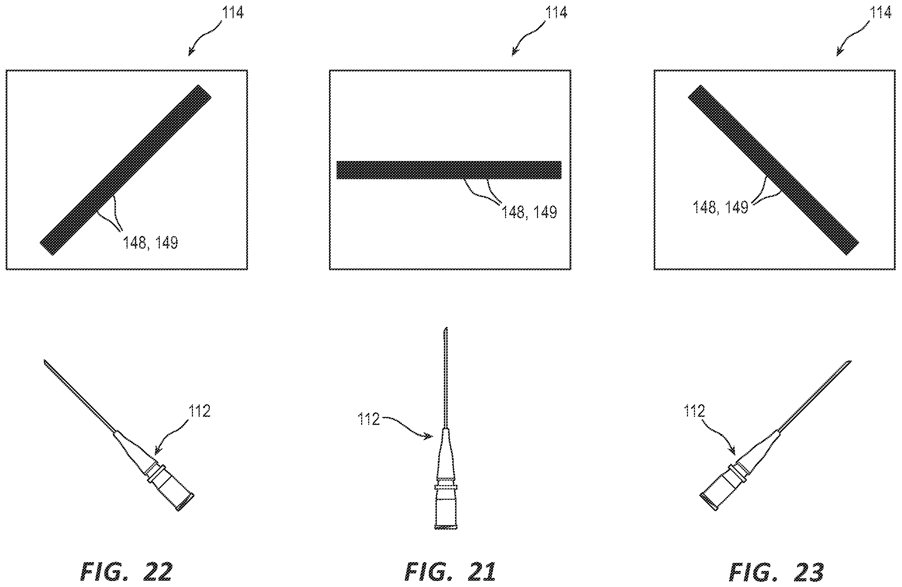

Optionally, a stand-alone optical interrogator 154 can be communicatively coupled to the console 102 by way of one of the ports 124. Alternatively, the console 102 can include an integrated optical interrogator integrated into the console 102. Such an optical interrogator is configured to emit input optical signals into a companion optical-fiber stylet 156 for shape sensing with the ultrasound-imaging system 100, which optical-fiber stylet 156, in turn, is configured to be inserted into a lumen of a medical device such as the needle 112 and convey the input optical signals from the optical interrogator 154 to a number of FBG sensors along a length of the optical-fiber stylet 156. The optical interrogator 154 is also configured to receive reflected optical signals conveyed by the optical-fiber stylet 156 reflected from the number of FBG sensors, the reflected optical signals indicative of a shape of the optical-fiber stylet 156. The optical interrogator 154 is also configured to convert the reflected optical signals into corresponding electrical signals for processing by the console 102 into distance and orientation information with respect to the target for dynamically adjusting a distance of the activated ultrasonic transducers 149, an orientation of the activated ultrasonic transducers 149, or both the distance and the orientation of the activated ultrasonic transducers 149 with respect to the target or the medical device when it is brought into proximity of the target. For example, the distance and orientation of the activated ultrasonic transducers 149 can be adjusted with respect to a blood vessel as the target. Indeed, an image plane can be established by the activated ultrasonic transducers 149 being perpendicular or parallel to the blood vessel in accordance with an orientation of the blood vessel. In another example, when a medical device such as the needle 112 is brought into proximity of the ultrasound probe 106, an image plane can be established by the activated ultrasonic transducers 149 being perpendicular to a medical-device plane including the medical device as shown in FIGS. 11-13 and 21-23 or parallel to the medical-device plane including the medical device for accessing the target with the medical device. The image plane can be perpendicular to the medical-device plane upon approach of the medical device and parallel to the medical-device plane upon insertion of the medical device (e.g., percutaneous puncture with the needle 112). The distance and orientation information can also be used for displaying an iconographic representation of the medical device on the display.

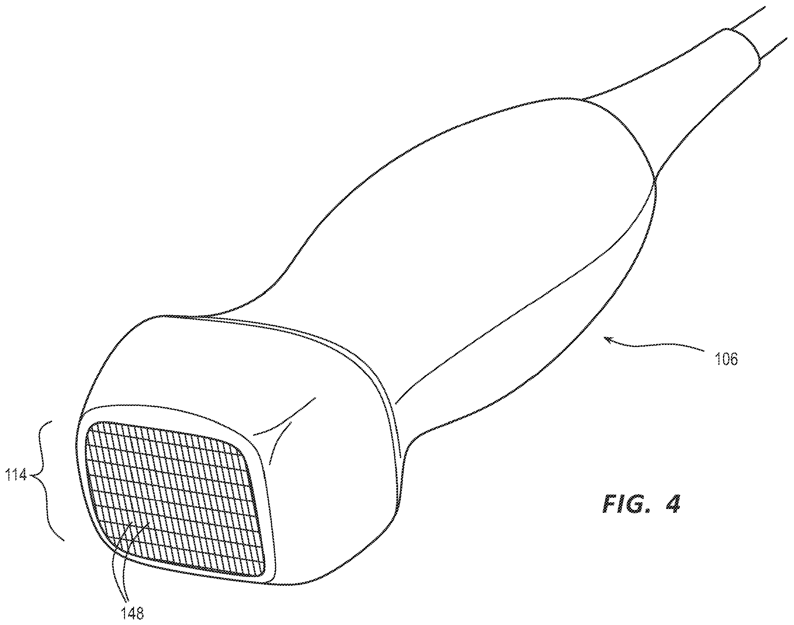

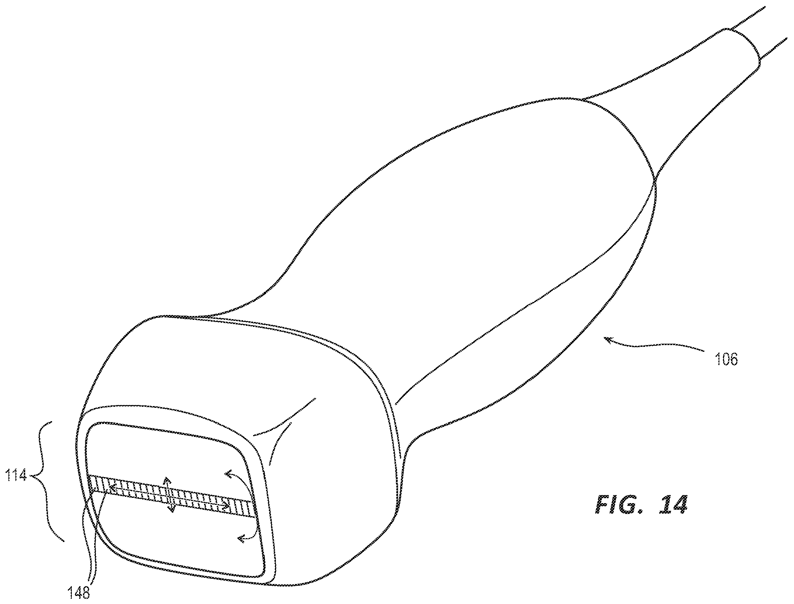

FIG. 4 illustrates the ultrasound probe 106 of the ultrasound-imaging system 100 configured as a 2-D ultrasound probe 106 in accordance with some embodiments. FIG. 14 illustrates the ultrasound probe 106 of the ultrasound-imaging system 100 configured as a linear ultrasound probe 106 in accordance with some embodiments.

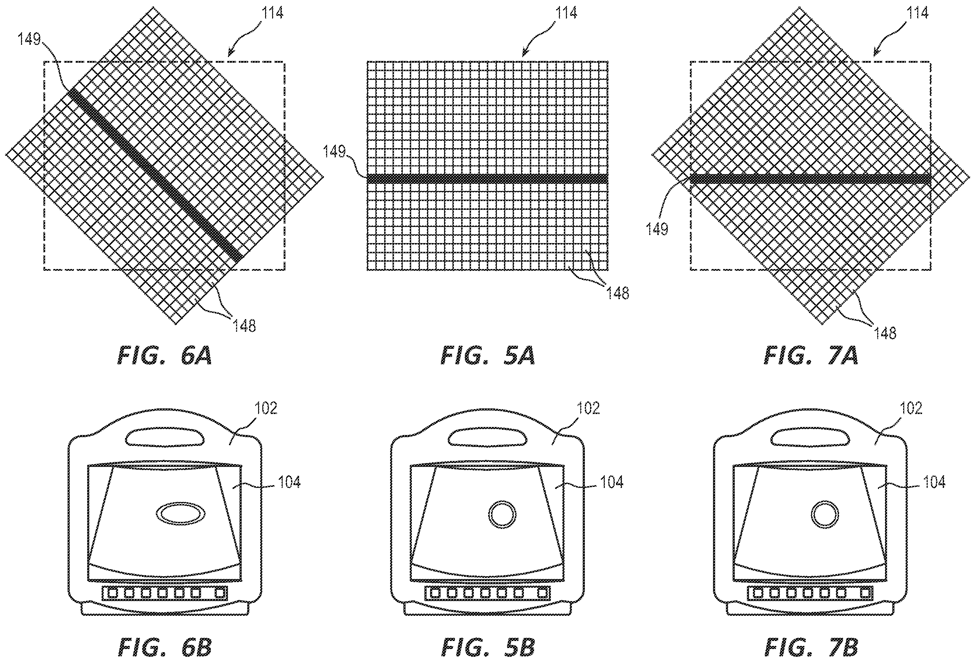

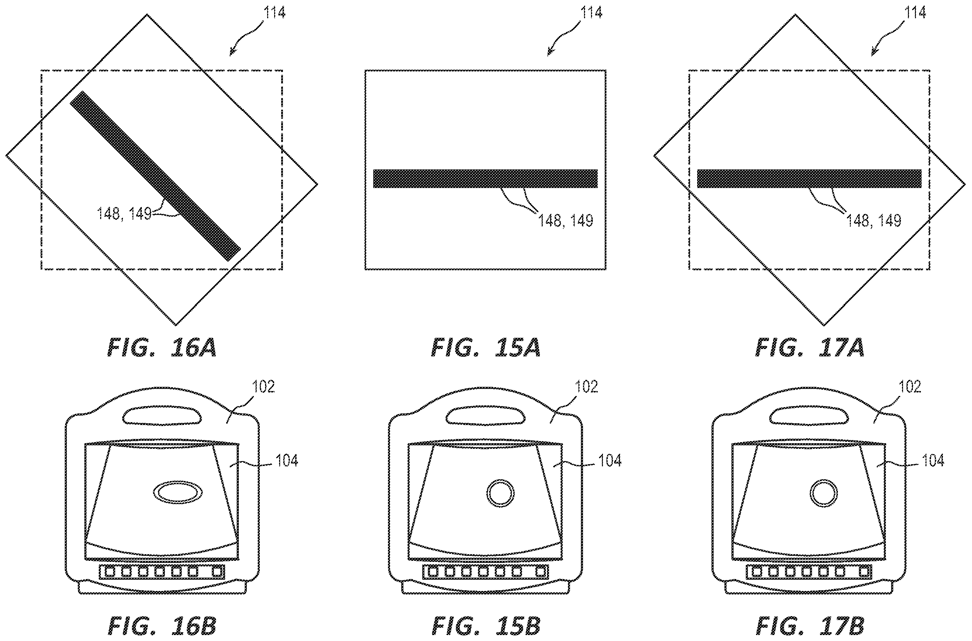

The ultrasound probe 106 includes a probe head 114 that houses a mounted and moveable (e.g., translatable or rotatable along a central axis) linear array of the ultrasonic transducers 148 or a 2-D array of the ultrasonic transducers 148, wherein the ultrasonic transducers 148 are piezoelectric transducers or capacitive micromachined ultrasonic transducers ("CMUTs"). When the ultrasound probe 106 is configured with the 2-D array of the ultrasonic transducers 148, a subset of the ultrasonic transducers 148 is linearly activated as needed for ultrasound imaging in accordance with ultrasound-imaging data, magnetic-field data, shape-sensing data, or a combination thereof to maintain the target in an image plane or switch to a different image plane (e.g., from perpendicular to a medical-device plane to parallel to the medical-device plane) including the target. *(See,* for example, the activated ultrasonic transducers 149 of FIGS. 5A, 7A, 10A, 12, or 13.) When the ultrasound probe 106 is configured with the moveable linear array of the ultrasonic transducers 148, the ultrasonic transducers 148 already activated for ultrasound imaging (e.g., a subset of the ultrasonic transducers 148 up to all the ultrasonic transducers 148) are moved together on the moveable linear array as needed for ultrasound imaging in accordance with ultrasound-imaging data, magnetic-field data, shape-sensing data, or a combination thereof to maintain the target in an image plane established by the activated ultrasonic transducers 149 or switch to a different image plane including the target. (*See,* for example, the activated ultrasonic transducers 149 of FIGS. 15A, 17A, 20A, 22, or 23.)

The probe head 114 is configured for placement against skin of the patient *P* proximate a prospective needle-insertion site where the activated ultrasonic transducers 149 in the probe head 114 can generate and emit the generated ultrasound signals into the patient *P* in a number of pulses, receive reflected ultrasound signals or ultrasound echoes from the patient *P* by way of reflection of the generated ultrasonic pulses by the body of the patient *P,* and convert the reflected ultrasound signals into corresponding electrical signals for processing into ultrasound images by the console 102 to which the ultrasound probe 106 is communicatively coupled. In this way, a clinician can employ the ultrasound-imaging system 100 to determine a suitable insertion site and establish vascular access with the needle 112 or another medical device.

The ultrasound probe 106 further includes the control buttons 110 for controlling certain aspects of the ultrasound-imaging system 100 during an ultrasound-based medical procedure, thus eliminating the need for the clinician to reach out of a sterile field around the patient P to control the ultrasound-imaging system 100. For example, a control button of the control buttons 110 can be configured to select or lock onto the target (e.g., a blood vessel, an organ, etc.) when pressed for visualization of the target in preparation for inserting the needle 112 or another medical device into the target. Such a control button can also be configured to deselect the target, which is useful whether the target was selected by the control button or another means such as by holding the ultrasound probe 106 stationary over the target to select the target, issuing a voice command to select the target, or the like.

FIG. 2 shows that the ultrasound probe 106 further includes a button and memory controller 138 for governing button and ultrasound probe 106 operation. The button and memory controller 138 can include non-volatile memory (e.g., EEPROM). The button and memory controller 138 is in operable communication with a probe interface 140 of the console 102, which includes an input/output ("I/O") component 142 for interfacing with the ultrasonic transducers 148 and a button and memory I/O component 144 for interfacing with the button and memory controller 138.

Also as seen in FIGS. 2 and 3A, the ultrasound probe 106 can include a magnetic-sensor array 146 for detecting a magnetized medical device such as the needle 112 during ultrasound-based medical procedures. The magnetic-sensor array 146 includes a number of magnetic sensors 150 embedded within or included on a housing of the ultrasound probe 106. The magnetic sensors 150 are configured to detect a magnetic field or a disturbance in a magnetic field as magnetic signals associated with the magnetized medical device when it is in proximity to the magnetic-sensor array 146. The magnetic sensors 150 are also configured to convert the magnetic signals from the magnetized medical device (e.g., the needle 112) into electrical signals for the console 102 to process into distance and orientation information for the magnetized medical device with respect to the predefined target, as well as for display of an iconographic representation of the magnetized medical device on the display screen 104. (*See* the magnetic field **B** of the needle 112 in FIG. 3A.) Thus, the magnetic-sensor array 146 enables the ultrasound-imaging system 100 to track the needle 112 or the like.

Though configured here as magnetic sensors, it is appreciated that the magnetic sensors 150 can be sensors of other types and configurations. Also, though they are described herein as included with the ultrasound probe 106, the magnetic sensors 150 of the magnetic-sensor array 146 can be included in a component separate from the ultrasound probe 106 such as a sleeve into which the ultrasound probe 106 is inserted or even a separate handheld device. The magnetic sensors 150 can be disposed in an annular configuration about the probe head 114 of the ultrasound probe 106, though it is appreciated that the magnetic sensors 150 can be arranged in other configurations, such as in an arched, planar, or semi-circular arrangement.

Each magnetic sensor of the magnetic sensors 150 includes three orthogonal sensor coils for enabling detection of a magnetic field in three spatial dimensions. Such 3-dimensional ("3-D") magnetic sensors can be purchased, for example, from Honeywell Sensing and Control of Morristown, NJ. Further, the magnetic sensors 150 are configured as Hall-effect sensors, though other types of magnetic sensors could be employed. Further, instead of 3-D sensors, a plurality of 1-dimensional ("1-D") magnetic sensors can be included and arranged as desired to achieve 1-, 2-, or 3-D detection capability.

Five magnetic sensors 150 are included in the magnetic-sensor array 146 so as to enable detection of a magnetized medical device such as the needle 112 in three spatial dimensions (e.g., X, Y, Z coordinate space), as well as the pitch and yaw orientation of the magnetized medical device itself. Detection of the magnetized medical device in accordance with the foregoing when the magnetized medical device is brought into proximity of the ultrasound probe 106 allows for dynamically adjusting a distance of the activated ultrasonic transducers 149, an orientation of the activated ultrasonic transducers 149, or both the distance and the orientation of the activated ultrasonic transducers 149 with respect to the target or the magnetized medical device. For example, the distance and orientation of the activated ultrasonic transducers 149 can be adjusted with respect to a blood vessel as the target. Indeed, an image plane can be established by the activated ultrasonic transducers 149 being perpendicular or parallel to the blood vessel in accordance with an orientation of the blood vessel. In another example, as shown among FIGS. 11-13 and 21-23, when the magnetized medical device is brought into proximity of the ultrasound probe 106, an image plane can be established by the activated ultrasonic transducers 149 being perpendicular to a medical-device plane including the magnetized medical device for accessing the target with the magnetized medical device. While not shown, the image plane can also be established by the activated ultrasonic transducers 149 being parallel to the medical-device plane including the magnetized medical device for accessing the target with the magnetized medical device such as after insertion of the medical device into the patient. Note that in some embodiments, orthogonal sensing components of two or more of the magnetic sensors 150 enable the pitch and yaw attitude of the magnetized medical device to be determined, which enables tracking with relatively high accuracy. In other embodiments, fewer than five or more than five magnetic sensors of the magnetic sensors 150 can be employed in the magnetic-sensor array 146. More generally, it is appreciated that the number, size, type, and placement of the magnetic sensors 150 of the magnetic-sensor array 146 can vary from what is explicitly shown here.

As shown in FIG. 2, the ultrasound probe 106 can further include an inertial measurement unit ("IMU") 158 or any one or more components thereof for inertial measurement selected from an accelerometer 160, a gyroscope 162, and a magnetometer 164 configured to provide positional-tracking data of the ultrasound probe 106 to the console 102 for stabilization of an image plane. The processor 116 is further configured to execute the logic 120 for processing the positional-tracking data for adjusting the distance of the activated ultrasonic transducers 149 from the target, the orientation of the activated ultrasonic transducers 149 to the target, or both the distance and the orientation of the activated ultrasonic transducers 149 with respect to the target to maintain the distance and the orientation of the activated ultrasonic transducers 149 with respect to the target when the ultrasound probe 106 is inadvertently moved with respect to the target.

It is appreciated that a medical device of a magnetizable material enables the medical device (e.g., the needle 112) to be magnetized by a magnetizer, if not already magnetized, and tracked by the ultrasound-imaging system 100 when the magnetized medical device is brought into proximity of the magnetic sensors 150 of the magnetic-sensor array 146 or inserted into the body of the patient P during an ultrasound-based medical procedure. Such magnetic-based tracking of the magnetized medical device assists the clinician in placing a distal tip thereof in a desired location, such as in a lumen of a blood vessel, by superimposing a simulated needle image representing the real-time distance and orientation of the needle 112 over an ultrasound image of the body of the patient *P* being accessed by the magnetized medical device. Such a medical device can be stainless steel such as SS 304 stainless steel; however, other suitable needle materials that are capable of being magnetized can be employed. So configured, the needle 112 or the like can produce a magnetic field or create a magnetic disturbance in a magnetic field detectable as magnetic signals by the magnetic-sensor array 146 of the ultrasound probe 106 so as to enable the distance and orientation of the magnetized medical device to be tracked by the ultrasound-imaging system 100 for dynamically adjusting the distance of the activated ultrasonic transducers 149, an orientation of the activated ultrasonic transducers 149, or both the distance and the orientation of the activated ultrasonic transducers 149 with respect to the magnetized medical device.

During operation of the ultrasound-imaging system 100, the probe head 114 of the ultrasound probe 106 is placed against skin of the patient P. An ultrasound beam 152 is produced so as to ultrasonically image a portion of a target such as a blood vessel beneath a surface of the skin of the patient *P.* (*See* FIG. 3A.) The ultrasonic image of the blood vessel can be depicted and stabilized on the display screen 104 of the ultrasound-imaging system 100 as shown in FIG. 3B despite inadvertent movements of the ultrasound probe 106. Indeed, this is shown among FIGS. 5A, 5B, 7A, 7B, 8A, 8B, 10A, and 10B for the ultrasound probe 106 configured with the 2-D array of the ultrasonic transducers 148 and FIGS. 15A, 15B, 17A, 17B, 18A, 18B, 20A, and 20B for the ultrasound probe 106 configured with the moveable linear array of the ultrasonic transducers 148.

FIGS. 5A and 5B illustrate the activated ultrasonic transducers 149 of the 2-D array of the ultrasonic transducers 148 of the ultrasound probe 106 in accordance with some embodiments. FIGS. 15A and 15B illustrate the activated ultrasonic transducers 149 of the moveable linear array of the ultrasonic transducers 148 of the ultrasound probe 106 in accordance with some embodiments. As shown in FIG. 7A, upon rotating the ultrasound probe 106 as might occur with an inadvertent movement of the ultrasound probe 106, dynamic adjustment of the activated ultrasonic transducers 149 occurs to maintain the target in the image plane. Such dynamic adjustment includes deactivating certain ultrasonic transducers and activating certain other ultrasonic transducers to maintain a distance and orientation of the activated ultrasonic transducers 149 to the target, which stabilizes the ultrasound image as shown in FIG. 7B. *(Compare* FIG. 7B *with* 5B.) Without such dynamic adjustment as shown by FIG. 6A, the distance and orientation of the activated ultrasonic transducers 149 to the target is not maintained, which results in a different ultrasound image as shown in FIG. 6B. *(Compare* FIG. 6B *with* 5B.) Likewise, as shown in FIG. 17A, upon rotating the ultrasound probe 106 as might occur with an inadvertent movement of the ultrasound probe 106, dynamic adjustment of the activated ultrasonic transducers 149 occurs to maintain the target in the image plane. Such dynamic adjustment includes automatically rotating the moveable linear array of the ultrasonic transducers 148 (within the probe head 114) to maintain a distance and orientation of the activated ultrasonic transducers 149 to the target, which stabilizes the ultrasound image as shown in FIG. 17B. *(Compare* FIG. 17B *with* 15B.) Without such dynamic adjustment as shown by FIG. 16A, the distance and orientation of the activated ultrasonic transducers 149 to the target is not maintained, which results in a different ultrasound image as shown in FIG. 16B. *(Compare* FIG. 16B *with* 15B.)

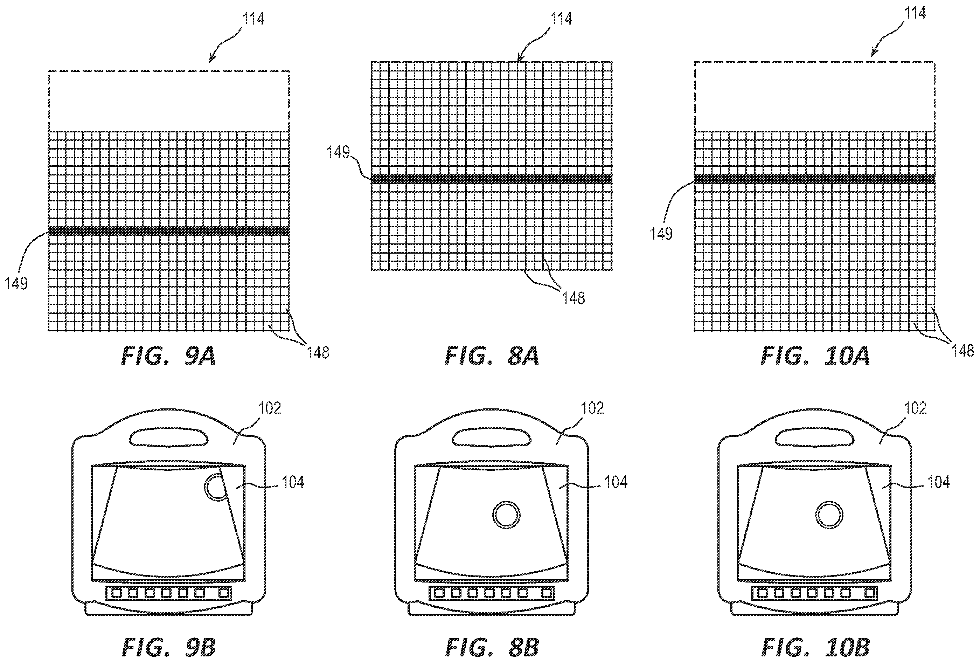

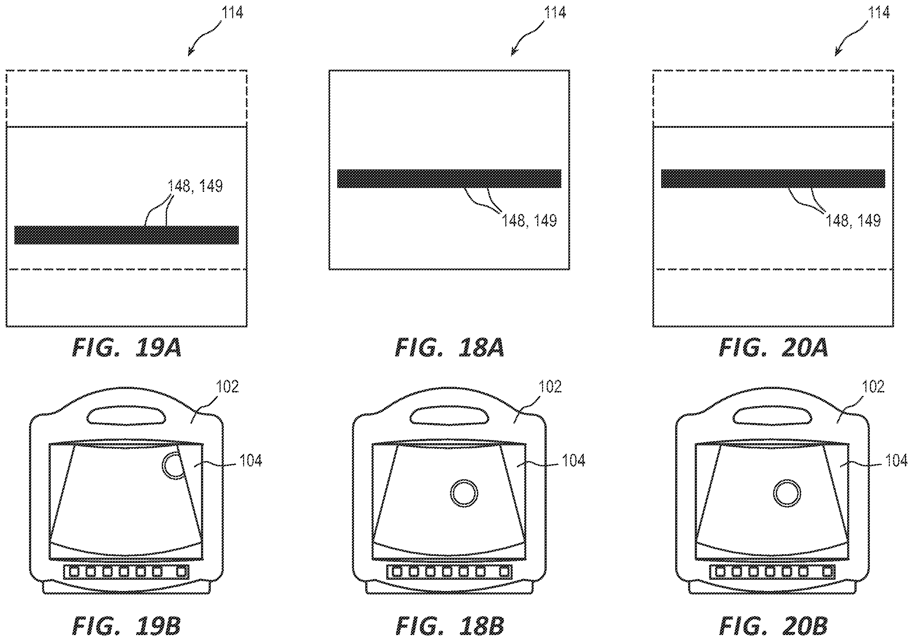

FIGS. 8A and 8B illustrate the activated ultrasonic transducers 149 of the 2-D array of the ultrasonic transducers 148 of the ultrasound probe 106 in accordance with some embodiments. FIGS. 18A and 18B illustrate the activated ultrasonic transducers 149 of the moveable linear array of the ultrasonic transducers 148 of the ultrasound probe 106 in accordance with some embodiments. As shown in FIG. 10A, upon translating the ultrasound probe 106 as might occur with an inadvertence movement of the ultrasound probe 106, dynamic adjustment of the activated ultrasonic transducers 149 occurs to maintain the target in the image plane. Such dynamic adjustment includes deactivating certain ultrasonic transducers and activating certain other ultrasonic transducers to maintain a distance and orientation of the activated ultrasonic transducers 149 to the target, which stabilizes the ultrasound image as shown in FIG. 10B. *(Compare* FIG. 10B *with* 8B.) Without such dynamic adjustment as shown by FIG. 9A, the distance and orientation of the activated ultrasonic transducers 149 to the target is not maintained, which results in a different ultrasound image as shown in FIG. 9B. *(Compare* FIG. 9B *with* 8B.) Likewise, as shown in FIG. 20A, upon translating the ultrasound probe 106 as might occur with an inadvertent movement of the ultrasound probe 106, dynamic adjustment of the activated ultrasonic transducers 149 occurs to maintain the target in the image plane. Such dynamic adjustment includes automatically translating the moveable linear array of the ultrasonic transducers 148 (within the probe head 114) to maintain a distance and orientation of the activated ultrasonic transducers 149 to the target, which stabilizes the ultrasound image as shown in FIG. 20B. *(Compare* FIG. 20B *with* 18B.) Without such dynamic adjustment as shown by FIG. 19A, the distance and orientation of the activated ultrasonic transducers 149 to the target is not maintained, which results in a different ultrasound image as shown in FIG. 19B. *(Compare* FIG. 19B *with* 18B.)

The ultrasound-imaging system 100 is configured to detect the distance and orientation of a medical device by way of the magnetic sensors 150 or shape-sensing optical-fiber stylet 156. By way of example, the magnetic-sensor array 146 of the ultrasound probe 106 is configured to detect a magnetic field of the magnetized medical device or a disturbance in a magnetic field due to the magnetized magnetic device. Each magnetic sensor of the magnetic sensors 150 in the magnetic-sensor array 146 is configured to spatially detect the needle 112 in 3-dimensional space. *(See* FIG. 3A.) Thus, during operation of the ultrasound-imaging system 100, magnetic field strength data of the medical device's magnetic field sensed by each magnetic sensor of the magnetic sensors 150 is forwarded to the processor 116 of the console 102, which computes in real-time the distance and orientation of the magnetized medical device useful for dynamically adjusting a distance of activated ultrasonic transducers 149, an orientation of the activated ultrasonic transducers 149, or both the distance and the orientation of the activated ultrasonic transducers 149 with respect to the magnetized medical device. Again, the distance and orientation of the magnetized medical device is also for graphical display on the display screen 104.

The distance or orientation of any point along an entire length of the magnetized medical device in a coordinate space with respect to the magnetic-sensor array 146 can be determined by the ultrasound-imaging system 100 using the magnetic-field strength data sensed by the magnetic sensors 150. Moreover, a pitch and yaw of the needle 112 can also be determined. Suitable circuitry of the ultrasound probe 106, the console 102, or other components of the ultrasound-imaging system 100 can provide the calculations necessary for such distance or orientation. In some embodiments, the needle 112 can be tracked using the teachings of one or more patents of U.S. Patent Nos.: 5,775,322; 5,879,297; 6,129,668; 6,216,028; and 6,263,230.

The distance and orientation information determined by the ultrasound-imaging system 100, together with an entire length of the magnetized medical device, as known by or input into the ultrasound-imaging system 100, enables the ultrasound-imaging system 100 to accurately determine the distance and orientation of the entire length of the magnetized medical device, including a distal tip thereof, with respect to the magnetic-sensor array 146. This, in turn, enables the ultrasound-imaging system 100 to superimpose an image of the needle 112 on an ultrasound image produced by the ultrasound beam 152 of the ultrasound probe 106 on the display screen 104, as well as dynamically adjusting the activated ultrasonic transducers 149. For example, the ultrasound image depicted on the display screen 104 can include depiction of the surface of the skin of the patient *P* and a subcutaneous blood vessel thereunder to be accessed by the needle 112, as well as a depiction of the magnetized medical device as detected by the ultrasound-imaging system 100 and its orientation to the vessel. The ultrasound image corresponds to an image acquired by the ultrasound beam 152 of the ultrasound probe 106. It should be appreciated that only a portion of an entire length of the magnetized medical device is magnetized and, thus, tracked by the ultrasound-imaging system 100.

Note that further details regarding structure and operation of the ultrasound-imaging system 100 can be found in U.S. Patent No. 9,456,766, titled "Apparatus for Use with Needle Insertion Guidance System."

In some instances, it may be advantageous for the ultrasound-imaging system 100 to determine an orientation of a target blood vessel and to establish an ultrasound image plane that is oriented perpendicular to the blood vessel as shown in FIG. 3B above among others. In some instances, the ultrasound probe 106, upon initial placement on the patient, may be oriented with respect to the patient such that the ultrasound probe 106, including the ultrasound image plane, is disposed at an angle with respect to the target blood vessel. For example, the probe 106, including the image plane, may be rotated out of alignment with the target blood vessel as illustrated in FIGS. 6A, 16A such that the shape of the blood vessel in the ultrasound image is elliptical. As such, it may be advantageous for the ultrasound-imaging system 100 to automatically detect the misalignment of the ultrasound probe 106 with respect the target blood vessel so that the probe 106 may manually re-oriented by the clinician or so that the 149... may be automatically adjusted to establish orient the ultrasound image plane in perpendicular alignment with the target blood vessel.

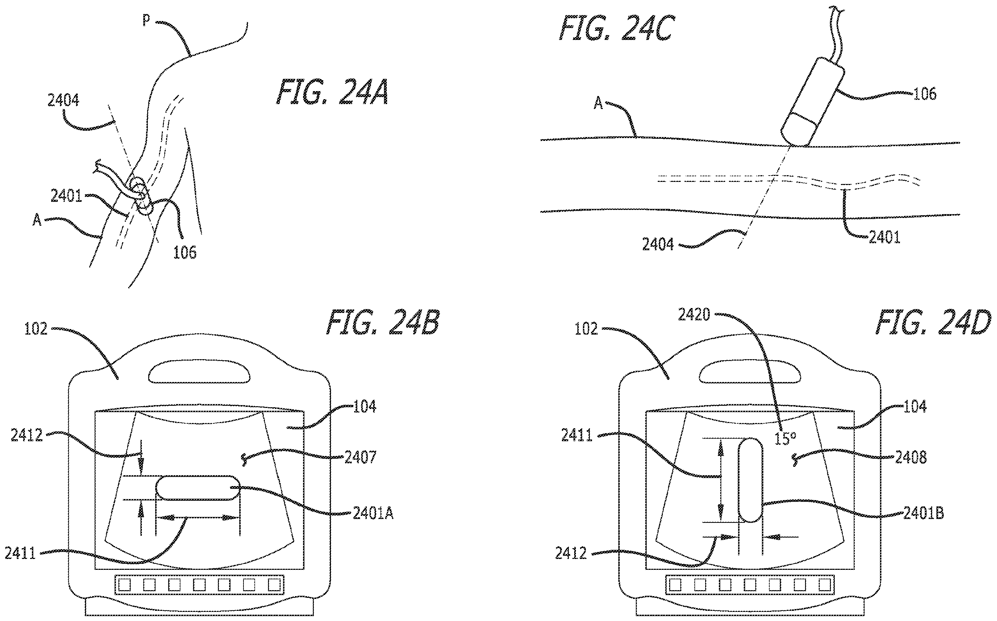

FIG. 24A illustrates a top view of the ultrasound probe 106 in a first instance where the ultrasound probe 106 is rotated (e.g., about a longitudinal axis of the ultrasound probe 106) with respect to the arm A of the patient P. As such, the image plane 2404 is disposed in a first misaligned orientation with respect to the target vein 2401, i.e., the image plane 2404 is not disposed in a perpendicular orientation with respect to the target vein 2401.

FIG. 24B illustrates an image of a target vein image 2401A as may be depicted in the ultrasound image 2407 in accordance with the first instance of misalignment of FIG. 24A. As a result of the first instance of misalignment of the image plane 2404, a 2-D target vein image 2401A of the target vein 2401 has a horizontally oriented elliptical shape in the ultrasound image 2407.

FIG. 24C illustrates a side view of the ultrasound probe 106 in a second instance where the ultrasound probe 106 is tilted with respect to the arm A of the patient P. As such, the image plane 2404 is disposed in a second misaligned orientation with respect to the target vein 2401, i.e., the image plane 2404 is gain not disposed in a perpendicular orientation with respect to the target vein 2401.

FIG. 24D illustrates an image of a target vein image 2401B as may be depicted in the ultrasound image 2408 in accordance with the second instance of misalignment of FIG. 24C. As a result of the second instance of misalignment of the image plane 2404, the 2-D target vein image 2401B of the target vein 2401 has a vertically oriented elliptical shape in the ultrasound image 2408.

In the first and second misalignment instances of the FIGS. 24A-24D, and combinations thereof, the target vein images 2401A, 2401B each define a length 2411 and a width 2412 of the elliptical shape. In the illustrated embodiment, the logic 120 detects the length 2411 and the width 2412 and calculates a parameter related to the misalignment of the image plane 2404. In some embodiments, the parameter may be a ratio of the length 2411 versus the width 2412 (e.g., the length 2411 divided by the width 2412). In other embodiments, parameter may be any other arithmetical calculation of the length 2411 and the width 2412, such as a difference between the length 2411 and the width 2412, for example. The logic 120 may then compare the calculated parameter with a parameter limit stored in memory and as a result of the comparison, determine that the ultrasound image plane is misaligned (i.e., not oriented perpendicular) with respect to the target vein 2401 when the calculated parameter exceeds the parameter limit in memory.

In some embodiments, the logic 120 may provide a notification to the clinician regarding the status of alignment. The notification may be audible, tactile, and/or visual. In some embodiments, the notification may indicate a magnitude of misalignment. For example, an audible notification may change volume or pitch in accordance with the magnitude of misalignment. By way of another example, a visual notification may include an indicium 2420 superimposed on the ultrasound image, such as a calculated angular indication of misalignment. In some embodiments, the clinician may manipulate the orientation of the ultrasound probe 106 into alignment with the target vein 2401, i.e., so that the target vein image is round and/or so that the notification indicates sufficient alignment.

The logic 120 automatically rotationally aligns the ultrasound image with the target vein 2401. For example, as shown in FIG. 7A, upon detecting misalignment of the image plane 2404, the logic 120 may adjust the activated ultrasonic transducers 149 *(see* FIG. 7A) to rotationally align the image plane 2404 with the target vessel 2401. Such adjustment may include deactivating certain ultrasonic transducers and activating certain other ultrasonic transducers to establish an orientation of the activated ultrasonic transducers 149 with respect to the target vein 2401. Likewise, as shown in FIG. 17A, upon detecting misalignment of the ultrasound probe 106, the logic 120 may adjust the activated ultrasonic transducers 149 to rotationally align the image plane with the target vessel 2401. Such adjustment includes automatically rotating the moveable linear array of the ultrasonic transducers 148 (within the probe head 114, see FIG. 17B) to establish an orientation of the activated ultrasonic transducers 149 to the target vein 2401.

In some embodiments, the ultrasound-imaging system 100 may be configured to detect a 180-degree misalignment of the ultrasound probe 106/ image plane 2404 with respect to the target blood vessel. In some instances, the orientation of the needle with respect to the direction of blood flow with a blood vessel may be defined according to medical procedure. For example, an intravenous catheter is generally inserted in the direction of blood flow, i.e., toward the heart of the patient. As such, it may be advantageous for the system 100 to detect the direction of the blood flow within a target blood vessel before insertion of the needle.

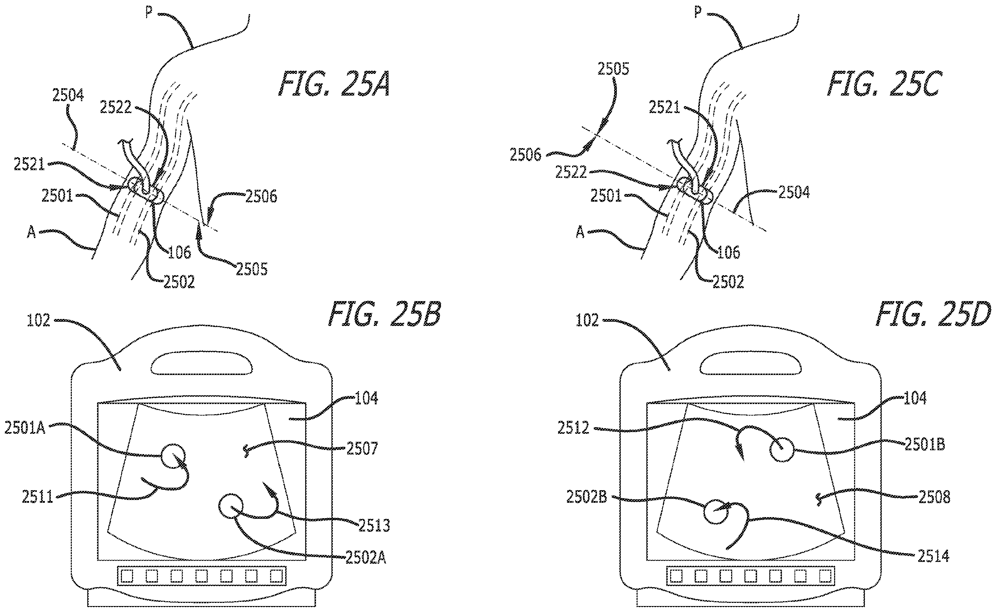

FIG. 25A illustrates a top view of the ultrasound probe 106 placed on the arm A of the patient P. Shown are a target vein 2501 and an artery 2502 extending along the arm A. According to a first instance, the ultrasound probe 106 is oriented with respect to the arm A of the patient P so that the front side 2521 of the probe 106 faces away from the patient P and the back side 2522 of the probe 106 faces toward the patient P. As such, the image plane 2504 is disposed in a first orientation with respect to the target vein 2501 and the artery 2502 so that a front side 2505 of the image plane 2504 faces upstream with respect to the blood flow within the target vein 2501 and downstream with respect to the blood flow within the artery 2502. Similarly, a back side 2506 of the image plane 2504 faces downstream with respect to the blood flow within the target vein 2501 and upstream with respect to the blood flow within the artery 2502. In some instances, the orientation of the ultrasound probe 106 and the resulting image plane 2504 may be consistent with a medical procedure, such as the placement of the peripherally inserted central catheter (PICC).

FIG. 25B illustrates an ultrasound image 2507 of a target vein 2501 and the adjacent artery 2502 including the target vein image 2501A and the artery image 2502A. In some embodiments, the front side 2505 of the image plane 2504 may be consistent with a screen of the display 104. In other words, a view of the target vein image 2501A and the artery image 2502A on the display 104 is consistent with view the target vein 2501 and the adjacent artery 2502 from the front side 2505 of the image plane 2504. As such, target vein image 2501A is a downstream view of the target vein 2501 and artery image 2502A is an upstream view of the artery 2502. Said another way, the direction of blood flow with respect to the target vein image 2501A is into the screen of the display 104 and the direction of blood flow with respect to the artery image 2502A is out of the screen of the display 104.

FIGS. 25C, 25D are similar to the FIGS 25A, 25B except that the orientation of the ultrasound 106 in FIGS. 25C, 25D is flipped 180 degrees (opposite) with respect to the orientation of the ultrasound probe 106 in FIGS. 25A, 25B according to a second placement instance of the ultrasound probe 106. According to the second instance, the ultrasound probe 106 is oriented with respect to the arm A of the patient P so that the back side 2522 of the probe 106 faces away from the patient P and the front side 2521 of the probe 106 faces toward the patient P. As such, the image plane 2504 is disposed in a second orientation with respect to the target vein 2501 and the artery 2502 so that a back side 2506 of the image plane 2504 faces upstream with respect to the blood flow within the target vein 2501 and downstream with respect to the blood flow within the artery 2502. Similarly, a front side 2505 of the image plane 2504 faces downstream with respect to the blood flow within the target vein 2501 and upstream with respect to the blood flow within the artery 2502 target vein 2501. Further similarly, the direction of blood flow with respect to the target vein image 2501B is out of the screen of the display 104 and the direction of blood flow with respect to the artery image 2502B is into the screen of the display 104.

In the illustrated embodiment, the ultrasound probe 106 includes doppler ultrasound capability. As such, the ultrasound probe 106 may generate doppler ultrasound data pertaining to blood flow within blood vessels rendered in the ultrasound image, i.e., the target vein images 2501A, 2501B and the artery images 2502A, 2502B of FIGS. 25B and 25D. The logic 120 may determine, from the doppler ultrasound data, the direction and/or velocity of the blood flow within the target vein 2501 and the artery 2502. More specifically, the logic 120 may determine that the blood flow with respect to the target vein image 2501A in the FIG. 25A is directed into the screen of the display 104 and that the blood flow with respect to the artery image 2502A is directed out of the screen of the display 104. Similarly, the logic 120 may determine that the blood flow with respect to the target vein image 2501B in the FIG. 25B is directed out of the screen of the display 104 and that the blood flow with respect to the artery image 2502B is directed into the screen of the display 104.

Having the determined the direction of blood flow of the target vein 2501, the logic 120 may provide a notification to the clinician as to the direction of blood flow with respect to the target vein image 2501A with the ultrasound image 2504. For example, the logic 120 may superimpose an indicum 2511 atop the ultrasound image 2507 of FIG. 25B indicating blood flow directed into the screen of the display 104 consistent with the direction of the blood flow within the target vein 2501 from the front side of the ultrasound probe 106 toward the back side of the ultrasound probe 106. Similarly, the logic 120 may superimpose an indicum 2512 atop the ultrasound image 2508 of FIG. 25D indicating blood flow directed out of the screen. In similar fashion, the logic 120 may superimpose an indicum 2513 atop the ultrasound image 2507 of FIG. 25B indicating blood flow directed out of the screen and superimpose an indicum 2514 atop the ultrasound image 2508 of FIG. 25D indicating blood flow directed into the screen. Although the indicia 2512, 2513 are illustrated as arrows, the indicia may take any form suitable for indicating a direction of the blood flow, including colored indicia.

In some embodiments, the indicia may be linked with a desired condition of a medical procedure. For example, in an instance where the procedure includes insertion of the needle into a target vein in a downstream direction, an indicum may indicate that insertion of the needle is allowed when the logic 120 determines that the direction of the blood flow with respect to the target vein image is into the screen. Conversely, in the same instance, an indicum may indicate that insertion of the needle is not allowed when the logic 120 determines that the direction of the blood flow with respect to the target vein image is out of the screen.

With further reference to FIGS. 25A-25D, the logic 120 may be configured to differentiate a vein from an artery based on anatomical awareness such as a spatial awareness of a target blood vessel with respect to other blood vessels or anatomical elements. Similarly, the logic 120 may differentiate a target blood vessel from adjacent blood vessels based on anatomical awareness. In some embodiments, the anatomical awareness may include an awareness that the target vein 2501 is closer to the skin than the artery 2502. By way of example, a medical procedure may include insertion of a peripherally inserted central catheter needle (PICC) within a brachial vein. The logic may compare the ultrasound image 2507 of FIG. 25B with one or more ultrasound images of brachial veins, in accordance with PICC medical procedure, stored in memory 118, where the one or more ultrasound images include the spatial positioning of the brachial vein in relation to other anatomical elements such as the artery 2502. As a result of the comparison, the logic 20 may determine with a degree of confidence (e.g., a percent probability) that the target vein image 2501A is indeed an image of the brachial vein. By way of another example, a medical procedure may include insertion of a needle within a brachial vein in a downstream direction. The logic 120 may compare the image 2507 with the one or more ultrasound images stored in memory 118. As a result of the comparison, the logic 120 may determine with a degree of confidence that the direction of the blood flow with respect to target vein image 2501B is out of the screen. As such in some instances, the ultrasound image 2508 may be indicative that the target vein image 2501B rotated 180 degrees from a desired orientation of the target vein image (e.g., the target vein image 2501A of FIG. 25B). In some embodiments, in further response to the comparison, the logic 120 may superimpose indicia (e.g., the indicum 2512) atop the ultrasound image 2508 of FIG. 25D indicating the direction of blood flow with respect to the target vein image 2501B.

### Methods

Methods of the foregoing ultrasound-imaging systems include methods implemented in the ultrasound-imaging systems. For example, a method of the ultrasound-imaging system 100 includes a non-transitory CRM (e.g., EEPROM) having the logic 120 stored thereon that causes the ultrasound-imaging system 100 to perform a set of operations for ultrasound imaging when the logic 120 is executed by the processor 116 of the console 102. Such a method may generally include activating operations, adjusting operations, processing operations, and displaying operations.

The activating operations include activating the ultrasonic transducers of the array of the ultrasonic transducers 148 of the ultrasound probe 106 communicatively coupled to the console 102. With the activating operation, the ultrasonic transducers 148 emit generated ultrasound signals into the patient *P,* receive reflected ultrasound signals from the patient *P,* and convert the reflected ultrasound signals into corresponding electrical signals for processing into ultrasound images. The activating operations can include activating an approximately linear subset of the ultrasonic transducers 148 of a 2-D array of the ultrasonic transducers 148. Alternatively, the activating operations can include activating a subset of the ultrasonic transducers 148 up to all the ultrasonic transducers 148 in the movable linear array of the ultrasonic transducers 148.

The adjusting operations include adjusting (including dynamically adjusting) a distance of the activated ultrasonic transducers 149 from a predefined target or area, an orientation of the activated ultrasonic transducers 149 to the predefined target or area, or both the distance and the orientation of the activated ultrasonic transducers 149 with respect to the predefined target or area. For example, a dynamic adjusting operation can be in response to an orientation of a blood vessel, such as the predefined target. The adjusting operations include adjusting the orientation and/or distance of the activated ultrasonic transducers 149 with respect to the orientation of the blood vessel so as to establish an image plane by the activated ultrasonic transducers 149 perpendicular or parallel to the blood vessel.

The processing operations include processing the corresponding electrical signals of the ultrasound signals including doppler ultrasound signals into the ultrasound images.

The displaying operations include displaying images on the display 104 communicatively coupled to the console 102 including the ultrasound images.

The processing operations may further include determining a shape of a target blood vessel rendered within the ultrasound image. The determining may also include identifying a length and a width of an elliptical target blood vessel image and further include calculating a parameter related to a difference between the length and the width such as a ratio, for example. The processing operations may further include comparing the calculated parameter with a parameter limit stored in memory 118 and as a result of the comparison, the operations may further include providing notification that the calculated parameter exceeds the parameter limit (i.e., that the image plane is not sufficiently aligned perpendicular to the blood vessel). The notification may be visual, tactile, audible, or any combination thereof.

In some embodiments, the processing operations may include determining, from the calculated parameter, an angle of misalignment between the target blood vessel and the image plane. The adjusting operations may also include adjusting the orientation of the activated ultrasonic transducers 149 with respect to the orientation of the blood vessel so as to establish an image plane by the activated ultrasonic transducers 149 in alignment with the target blood vessel (i.e., perpendicular to the blood vessel) in response to the calculated parameter.

The processing operations may include differentiating a vein image from an artery image within the ultrasound image based on anatomical awareness such as a spatial awareness of a target blood vessel with respect to other blood vessels or anatomical elements. Similarly, the operations may include differentiating a target blood vessel from adjacent blood vessels based on anatomical awareness. In some embodiments, the logic 120 may compare target blood vessel image with one or more ultrasound images stored in memory 118. As a result of the comparison, the logic 120 may determine with a degree of confidence (e.g., a percent probability) that the target blood vessel image is indeed an image of target blood vessel based on anatomical spatial awareness the target blood vessel in relation to adjacent anatomical elements, such as blood vessels, bones, and the like. In some embodiments, the logic 120 may determine a direction of blood flow within the target blood vessel with respect to the ultrasound image of the target blood vessel based at least partially on the anatomical awareness of the target blood vessel.

The display operations may further include rendering an indicium on the display in combination with a blood vessel image identifying the blood vessel image as an image of the target blood vessel.

The processing operations may further include receiving doppler ultrasound data from the ultrasound probe 106 and processing the doppler ultrasound data to determine indicating a direction and/or velocity within the target blood vessel with respect to the ultrasound image plane. The display operations may then render an indicium on the display 104 in combination with the ultrasound image of the target blood vessel where the indicium indicates the direction of blood flow with respect to the target blood vessel image.

As to magnetic signal-related operations, the method can include a converting operation. The converting operation includes converting magnetic signals from a magnetized medical device (e.g., the needle 112) with the magnetic-sensor array 146 of the ultrasound probe 106 into corresponding electrical signals. The processing operations further include processing the corresponding electrical signals of the magnetic signals with the processor 116 into distance and orientation information with respect to the predefined target or area. The displaying operations further include displaying an iconographic representation of the medical device on the display screen 104.

The method may further include an adjusting operation in response to the magnetic signals. The adjusting operation includes adjusting the distance and orientation of the activated ultrasonic transducers 149 with respect to the predefined target or area when the medical device is brought into proximity of the ultrasound probe 106. The adjusting operation establishes an image plane by the activated ultrasonic transducers 149 perpendicular or parallel to the medical-device plane including the medical device for accessing the predefined target or area with the medical device. The establishing of the image plane can be perpendicular to the medical-device plane upon approach of the medical device and parallel to the medical-device plane upon insertion of the medical device. The image plane can include a blood vessel as the predefined target or area and the medical-device plane can include the needle 112 as the medical device.

The method may further include a number of optical signal-related operations in combination with further processing and displaying operations. The optical signal-related operations include emitting input optical signals, receiving reflected optical signals, and converting the reflected optical signals into corresponding electrical signals of the optical signals by the optical interrogator 154. The optical signal-related operations also include conveying the input optical signals from the optical interrogator 154 to the number of FBG sensors along the length of the optical-fiber stylet 156, as well as conveying the reflected optical signals from the number of FBG sensors back to the optical interrogator 154 with the optical-fiber stylet 156 disposed in a lumen of the medical device. The processing operation further include processing the corresponding electrical signals of the optical signals with the processor 116 into distance and orientation information with respect to the predefined target or area. The displaying operations further include displaying an iconographic representation of a medical device on the display 104.

The method may further include an adjusting operation in response to the optical signals. The adjusting operation includes adjusting the distance and orientation of the activated ultrasonic transducers 149 with respect to the predefined target or area when the medical device is brought into proximity of the ultrasound probe 106. The adjusting operation establishes an image plane by the activated ultrasonic transducers 149 perpendicular or parallel to the medical-device plane including the medical device for accessing the predefined target or area with the medical device. Again, the establishing of the image plane is perpendicular to the medical-device plane upon approach of the medical device and parallel to the medical-device plane upon insertion of the medical device. The image plane includes a blood vessel as the predefined target or area and the medical-device plane includes the needle 112 as the medical device.

The method can further include a data-providing operation in combination with further processing operations. The data-providing operation includes providing positional-tracking data to the console 102 from the accelerometer 160, the gyroscope 162, the magnetometer 164, or a combination thereof of the ultrasound probe 106. The processing operations further include processing the positional-tracking data with the processor 116 for the adjusting operation.

The method can further include a maintaining operation. The maintaining operation includes maintaining the distance and the orientation of the activated ultrasonic transducers 149 with respect to the predefined target or area when the ultrasound probe 106 is inadvertently moved with respect to the predefined target or area.

While some particular embodiments have been disclosed herein, and while the particular embodiments have been disclosed in some detail, it is not the intention for the particular embodiments to limit the scope of the claims. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the appended claims.

## Claims

1. An ultrasound-imaging system (100), comprising:
an ultrasound probe (106) including an array of ultrasonic transducers (149), activated ultrasonic transducers of the array of ultrasonic transducers configured to emit generated ultrasound signals into a patient, receive reflected ultrasound signals from the patient, and convert the reflected ultrasound signals into corresponding electrical signals of the ultrasound signals for processing into ultrasound images;
a console (102) configured to communicate with the ultrasound probe, the console including one or more processors and a non-transitory computer-readable medium having stored thereon logic, which, when executed by the one or more processors, causes operations including:
defining an ultrasound image of a blood vessel in accordance with an image plane of the ultrasound probe;
determining a misalignment between the blood vessel and the image plane;
providing notification in response to determining the misalignment; and
adjusting an orientation of the activated ultrasonic transducers to orient the image plane perpendicular to the blood vessel.

2. The ultrasound-imaging system of claim 1, wherein the ultrasound image of a blood vessel defines an elliptical shape; and optionally wherein determining the misalignment includes:
identifying a length and a width of the elliptical shape;
calculating a parameter relating to a difference between the length and the width; and
comparing the calculated parameter with a parameter limit stored in memory.

3. The ultrasound-imaging system of any of the preceding claims, wherein the notification is tactile, audible, visual, or any combination thereof.

4. The ultrasound-imaging system of any of the preceding claims, wherein:
the ultrasound probe includes doppler ultrasound capability; and
the operations further include determining a direction of blood flow within the blood vessel with respect the image plane based on doppler ultrasound data; and
optionally wherein rendering the ultrasound image of the blood vessel includes superimposing an indicum atop the ultrasound image of the blood vessel, the indicum indicating the direction of the blood flow.

5. The ultrasound-imaging system of any of the preceding claims, wherein the operations further include:
comparing the ultrasound image of the blood vessel with one or more ultrasound images of blood vessels stored in memory, the one or more ultrasound images pertaining to a defined medical procedure; and
providing a notification when, as a result of the comparison, an orientation of the image plane of the ultrasound image of the blood vessel is determined to be opposite to an orientation of a corresponding image plane of the one or more ultrasound images; and optionally wherein comparing the ultrasound image includes comparing the spatial positioning of the blood vessel in relation to adjacent anatomical elements in the ultrasound image of the blood vessel with the spatial positioning of a corresponding blood vessel in relation to corresponding adjacent anatomical elements in the one or more ultrasound images.

6. The ultrasound-imaging system of any of the preceding claims, the ultrasound probe further comprising: an array (146) of magnetic sensors (150) configured to convert magnetic signals from a magnetized medical device into corresponding electrical signals of the magnetic signals for processing by the processor into distance and orientation information with respect to the blood vessel for display of an iconographic representation of the medical device on the display screen; and optionally wherein the distance and orientation of the activated ultrasonic transducers is adjusted with respect to the blood vessel when the medical device is brought into proximity of the ultrasound probe, a device image plane established by the activated ultrasonic transducers being perpendicular or parallel to a medical-device plane including the medical device for accessing the blood vessel with the medical device.

7. The ultrasound-imaging system of claim 6, further comprising:
a stand-alone optical interrogator (154) communicatively coupled to the console or an integrated optical interrogator integrated into the console, the optical interrogator configured to emit input optical signals, receive reflected optical signals, and convert the reflected optical signals into corresponding electrical signals of the optical signals for processing by the processor into distance and orientation information with respect to the blood vessel for display of an iconographic representation of the medical device on the display; and
an optical-fiber stylet (156) configured to convey the input optical signals from the optical interrogator to a number of fiber Bragg grating, FBG, sensors along a length of the optical-fiber stylet and the reflected optical signals from the number of FBG sensors back to the optical interrogator, the optical-fiber stylet configured to be disposed in a lumen of the medical device.

8. The ultrasound-imaging system of any of the preceding claims, the ultrasound probe further comprising an accelerometer (160), a gyroscope (162), a magnetometer (164), or a combination thereof configured to provide positional-tracking data to the console, the processor further configured to execute the instructions for processing the positional-tracking data for the adjusting of the distance of the activated ultrasonic transducers from the blood vessel, the orientation of the activated ultrasonic transducers to the blood vessel, or both the distance and the orientation of the activated ultrasonic transducers with respect to the blood vessel; and/or, wherein the distance and the orientation of the activated ultrasonic transducers is maintained with respect to the blood vessel when the ultrasound probe is inadvertently moved with respect to the blood vessel; and/or wherein the operations further include rendering the ultrasound image of the blood vessel on a display (104) of the system.

9. A method of an ultrasound-imaging system (100) including a non-transitory computer-readable medium, CRM, having executable instructions that cause the ultrasound-imaging system to perform a set of operations for ultrasound imaging when the instructions are executed by a processor (116) of a console (102) of the ultrasound-imaging system, the method comprising:
activating ultrasonic transducers (149) of an array (148) of ultrasonic transducers of an ultrasound probe communicatively coupled to the console, whereby the ultrasonic transducers emit generated ultrasound signals into a patient, receive reflected ultrasound signals from the patient, and convert the reflected ultrasound signals into corresponding electrical signals of the ultrasound signals for processing into ultrasound images;
defining an ultrasound image of a blood vessel in accordance with an image plane of the ultrasound probe,
determining a misalignment between the blood vessel and the image plane;
providing notification in response to determining the misalignment; and
adjusting an orientation of the activated ultrasonic transducers to orient the image plane perpendicular to the blood vessel.

10. The method of claim 9, wherein the ultrasound image of a blood vessel defines an elliptical shape, and optionally wherein determining the misalignment comprises:
identifying a length and a width of the elliptical shape;
calculating a parameter relating to a difference between the length and the width; and
comparing the calculated parameter with a parameter limit stored in memory.

11. The method of any of claims 9 or 10, wherein the notification is tactile, audible, visual, or any combination thereof.

12. The method of any of claims 9-11, wherein the ultrasound probe includes doppler ultrasound capability, the method further comprising determining a direction of blood flow within the blood vessel with respect the image plane based on doppler ultrasound data; and optionally wherein rendering the ultrasound image of the blood vessel includes superimposing an indicum atop the ultrasound image of the blood vessel, the indicum indicating the direction of the blood flow.

13. The method of any of claims 9-12, further comprising:
comparing the ultrasound image of the blood vessel with one or more ultrasound images of blood vessels stored in memory, the one or more ultrasound images pertaining to a defined medical procedure; and
providing a notification when, as a result of the comparison, an orientation of the image plane of the ultrasound image of the blood vessel is determined to be opposite to the orientation of the image plane of the one or more ultrasound images; and optionally wherein comparing the ultrasound image includes comparing the spatial positioning of the blood vessel in relation to adjacent anatomical elements in the ultrasound image of the blood vessel with the spatial positioning of a corresponding blood vessel in relation to corresponding adjacent anatomical elements in the one or more ultrasound images.

14. The method of any of claims 9-13, further comprising:
converting magnetic signals from a magnetized medical device with an array of magnetic sensors of the ultrasound probe into corresponding electrical signals of the magnetic signals;
processing the corresponding electrical signals of the magnetic signals with the processor into distance and orientation information with respect to the blood vessel; and
rendering an iconographic representation of the medical device on the display (104); and the method optionally
1) further comprising:
emitting input optical signals, receiving reflected optical signals, and converting the reflected optical signals into corresponding electrical signals of the optical signals by a stand-alone optical interrogator (154) communicatively coupled to the console or an integrated optical interrogator integrated into the console;
conveying the input optical signals from the optical interrogator to a number of fiber Bragg grating,FBG, sensors along a length of an optical-fiber stylet (156) and the reflected optical signals from the number of FBG sensors back to the optical interrogator with the optical-fiber stylet disposed in a lumen of the medical device;
processing the corresponding electrical signals of the optical signals with the processor into distance and orientation information with respect to the blood vessel; and
rendering an iconographic representation of a medical device on the display (104); and/or
2) further comprising adjusting the distance and orientation of the activated ultrasonic transducers with respect to the blood vessel when the medical device is brought into proximity of the ultrasound probe, thereby establishing a device image plane by the activated ultrasonic transducers perpendicular or parallel to a medical-device plane including the medical device for accessing the blood vessel with the medical device.

15. The method of any of claims 9-14, further comprising:
providing positional-tracking data to the console from an accelerometer (160), a gyroscope (162), a magnetometer (164), or a combination thereof of the ultrasound probe; and
processing the positional-tracking data with the processor for the adjusting of the distance of the activated ultrasonic transducers from the blood vessel, the orientation of the activated ultrasonic transducers to the blood vessel, or both the distance and the orientation of the activated ultrasonic transducers with respect to the blood vessel; and/or further comprising maintaining the distance and the orientation of the activated ultrasonic transducers with respect to the blood vessel when the ultrasound probe is inadvertently moved with respect to the blood vessel; and/or further comprising rendering the ultrasound image of the blood vessel on a display (104) coupled with the console (102).

## Patentansprüche

1. Ultraschallbildgebungssystem (100), umfassend:
eine Ultraschallsonde (106), die ein Array von Ultraschallwandlern (149) einschließt, wobei aktivierte Ultraschallwandler des Arrays von Ultraschallwandlern ausgebildet sind, erzeugte Ultraschallsignale in einen Patienten zu emittieren, reflektierte Ultraschallsignale von dem Patienten zu empfangen und die reflektierten Ultraschallsignale in entsprechende elektrische Signale der Ultraschallsignale zur Verarbeitung zu Ultraschallbildern umzuwandeln;
eine Konsole (102), die ausgebildet ist, mit der Ultraschallsonde zu kommunizieren, wobei die Konsole einen oder mehrere Prozessoren und ein nicht transitorisches computerlesbares Medium einschließt, auf dem eine Logik gespeichert ist, die,
wenn von einem oder mehreren Prozessoren ausgeführt, Operationen veranlasst, einschließend:
Definieren eines Ultraschallbildes eines Blutgefäßes in Übereinstimmung mit einer Bildebene der Ultraschallsonde;
Bestimmen einer Fehlausrichtung zwischen dem Blutgefäß und der Bildebene;
Bereitstellen einer Signalisierung als Reaktion auf das Bestimmen der Fehlausrichtung; und
Anpassen einer Ausrichtung der aktivierten Ultraschallwandler, um die Bildebene senkrecht zu dem Blutgefäß auszurichten.

2. Ultraschallbildgebungssystem nach Anspruch 1, wobei das Ultraschallbild eines Blutgefäßes eine elliptische Form definiert; und wahlweise, wobei das Bestimmen der Fehlausrichtung Folgendes einschließt:
Identifizieren einer Länge und einer Breite der elliptischen Form;
Berechnen eines Parameters, der sich auf einen Unterschied zwischen der Länge und der Breite bezieht; und
Vergleichen des berechneten Parameters mit einem im Speicher gespeicherten Parametergrenzwert.

3. Ultraschallbildgebungssystem nach einem der vorstehenden Ansprüche, wobei die Signalisierung taktil, hörbar, visuell oder eine beliebige Kombination davon ist.

4. Ultraschallbildgebungssystem nach einem der vorstehenden Ansprüche, wobei:
die Ultraschallsonde eine Doppler-Ultraschall-Fähigkeit einschließt; und
die Operationen weiter das Bestimmen einer Richtung des Blutflusses innerhalb des Blutgefäßes in Bezug auf die Bildebene, basierend auf Doppler-Ultraschalldaten, einschließen; und wahlweise, wobei das Darstellen des Ultraschallbildes des Blutgefäßes, das Überlagern eines Anzeigeelements auf das Ultraschallbild des Blutgefäßes einschließt, wobei das Anzeigeelement die Richtung des Blutflusses anzeigt.

5. Ultraschallbildgebungssystem nach einem der vorstehenden Ansprüche, wobei die Operationen weiter Folgendes einschließen:
Vergleichen des Ultraschallbildes des Blutgefäßes mit einem oder mehreren im Speicher gespeicherten Ultraschallbildern von Blutgefäßen, wobei sich das eine oder die mehreren Ultraschallbilder auf ein definiertes medizinisches Verfahren beziehen; und
Bereitstellen einer Signalisierung, wenn als Ergebnis des Vergleichs bestimmt wird, dass eine Ausrichtung der Bildebene des Ultraschallbildes des Blutgefäßes entgegengesetzt zu einer Ausrichtung einer entsprechenden Bildebene des einen oder der mehreren Ultraschallbilder ist; und wahlweise, wobei das Vergleichen des Ultraschallbildes das Vergleichen der räumlichen Positionierung des Blutgefäßes in Bezug auf benachbarte anatomische Elemente in dem Ultraschallbild des Blutgefäßes mit der räumlichen Positionierung eines entsprechenden Blutgefäßes in Bezug auf entsprechende benachbarte anatomische Elemente in dem einen oder den mehreren Ultraschallbildern einschließt.

6. Ultraschallbildgebungssystem nach einem der vorstehenden Ansprüche, wobei die Ultraschallsonde weiter Folgendes umfasst: ein Array (146) von Magnetsensoren (150), das ausgebildet ist, magnetische Signale von einer magnetisierten medizinischen Vorrichtung in entsprechende elektrische Signale der magnetischen Signale zur Verarbeitung durch den Prozessor in Distanz- und Ausrichtungsinformationen in Bezug auf das Blutgefäß zur Anzeige einer ikonografischen Darstellung der medizinischen Vorrichtung auf dem Anzeigebildschirm umzuwandeln; und wahlweise, wobei die Distanz und Ausrichtung der aktivierten Ultraschallwandler in Bezug auf das Blutgefäß angepasst werden, wenn die medizinische Vorrichtung in die Nähe der Ultraschallsonde gebracht wird, wobei eine durch die aktivierten Ultraschallwandler hergestellte Bildebene der Vorrichtung senkrecht oder parallel zu einer Ebene der medizinischen Vorrichtung ist, die die medizinische Vorrichtung zum Zugreifen auf das Blutgefäß mit der medizinischen Vorrichtung einschließt.

7. Ultraschallbildgebungssystem nach Anspruch 6, weiter umfassend:
einen alleinstehenden optischen Interrogator (154), der kommunikativ mit der Konsole gekoppelt ist, oder einen integrierten optischen Interrogator, der in die Konsole integriert ist, wobei der optische Interrogator ausgebildet ist, optische Eingangssignale zu emittieren, reflektierte optische Signale zu empfangen und die reflektierten optischen Signale in entsprechende elektrische Signale der optischen Signale zur Verarbeitung durch den Prozessor in Distanz- und Ausrichtungsinformationen in Bezug auf das Blutgefäß zur Anzeige einer ikonografischen Darstellung der medizinischen Vorrichtung auf der Anzeige umzuwandeln; und
einen Lichtleitermandrin (156), der ausgebildet ist, die optischen Eingangssignale von dem optischen Interrogator zu einer Anzahl von Faser-Bragg-Gitter(FBG)-Sensoren entlang einer Länge des Lichtleitermandrins und die reflektierten optischen Signale von der Anzahl von FBG-Sensoren zurück zu dem optischen Interrogator zu leiten, wobei der Lichtleitermandrin ausgebildet ist, in einem Lumen der medizinischen Vorrichtung angeordnet zu sein.

8. Ultraschallbildgebungssystem nach einem der vorstehenden Ansprüche, wobei die Ultraschallsonde weiter Folgendes umfasst einen Beschleunigungsmesser (160), ein Gyroskop (162), ein Magnetometer (164) oder eine Kombination davon, die ausgebildet sind, Positionsverfolgungsdaten für die Konsole bereitzustellen, wobei der Prozessor weiter ausgebildet ist, die Anweisungen zur Verarbeitung der Positionsverfolgungsdaten für das Anpassen der Distanz der aktivierten Ultraschallwandler zu dem Blutgefäß, der Ausrichtung der aktivierten Ultraschallwandler zu dem Blutgefäß oder sowohl der Distanz als auch der Ausrichtung der aktivierten Ultraschallwandler in Bezug auf das Blutgefäß auszuführen; und/oder, wobei die Distanz und die Ausrichtung der aktivierten Ultraschallwandler in Bezug auf das Blutgefäß aufrechterhalten wird, wenn die Ultraschallsonde unbeabsichtigt in Bezug auf das Blutgefäß bewegt wird; und/oder wobei die Operationen weiter das Darstellen des Ultraschallbildes des Blutgefäßes auf einer Anzeige (104) des Systems einschließen.

9. Verfahren eines Ultraschallbildgebungssystems (100), das ein nicht transitorisches computerlesbares Medium, CRM, einschließt, das ausführbare Anweisungen aufweist, die das Ultraschallbildgebungssystem veranlassen, einen Satz von Operationen für Ultraschallbildgebung auszuführen, wenn die Anweisungen durch einen Prozessor (116) einer Konsole (102) des Ultraschallbildgebungssystems ausgeführt werden, wobei das Verfahren Folgendes umfasst:
Aktivieren von Ultraschallwandlern (149) eines Arrays (148) von Ultraschallwandlern einer Ultraschallsonde, die kommunikativ mit der Konsole gekoppelt ist, wobei die Ultraschallwandler erzeugte Ultraschallsignale in einen Patienten emittieren, reflektierte Ultraschallsignale von dem Patienten empfangen und die reflektierten Ultraschallsignale in entsprechende elektrische Signale der Ultraschallsignale zur Verarbeitung zu Ultraschallbildern umwandeln;
Definieren eines Ultraschallbildes eines Blutgefäßes in Übereinstimmung mit einer Bildebene der Ultraschallsonde,
Bestimmen einer Fehlausrichtung zwischen dem Blutgefäß und der Bildebene;
Bereitstellen einer Signalisierung als Reaktion auf das Bestimmen der Fehlausrichtung; und
Anpassen einer Ausrichtung der aktivierten Ultraschallwandler, um die Bildebene senkrecht zu dem Blutgefäß auszurichten.

10. Verfahren nach Anspruch 9, wobei das Ultraschallbild eines Blutgefäßes eine elliptische Form definiert, und wahlweise, wobei das Bestimmen der Fehlausrichtung Folgendes umfasst:
Identifizieren einer Länge und einer Breite der elliptischen Form;
Berechnen eines Parameters, der sich auf einen Unterschied zwischen der Länge und der Breite bezieht; und
Vergleichen des berechneten Parameters mit einem im Speicher gespeicherten Parametergrenzwert.

11. Verfahren nach einem der Ansprüche 9 oder 10, wobei die Signalisierung taktil, hörbar, visuell oder eine beliebige Kombination davon ist.

12. Verfahren nach einem der Ansprüche 9-11, wobei die Ultraschallsonde eine Doppler-Ultraschall-Fähigkeit einschließt, wobei das Verfahren weiter das Bestimmen einer Richtung des Blutflusses innerhalb des Blutgefäßes in Bezug auf die Bildebene, basierend auf Doppler-Ultraschalldaten, umfasst; und wahlweise, wobei das Darstellen des Ultraschallbildes des Blutgefäßes, das Überlagern eines Anzeigeelements auf das Ultraschallbild des Blutgefäßes einschließt, wobei das Anzeigeelement die Richtung des Blutflusses anzeigt.

13. Verfahren nach einem der Ansprüche 9-12, weiter umfassend:
Vergleichen des Ultraschallbildes des Blutgefäßes mit einem oder mehreren im Speicher gespeicherten Ultraschallbildern von Blutgefäßen, wobei sich das eine oder die mehreren Ultraschallbilder auf ein definiertes medizinisches Verfahren beziehen; und
Bereitstellen einer Signalisierung, wenn als Ergebnis des Vergleichs, bestimmt wird, dass eine Ausrichtung der Bildebene des Ultraschallbildes des Blutgefäßes entgegengesetzt zu der Ausrichtung der Bildebene des einen oder der mehreren Ultraschallbilder ist; und wahlweise, wobei das Vergleichen des Ultraschallbildes das Vergleichen der räumlichen Positionierung des Blutgefäßes in Bezug auf benachbarte anatomische Elemente in dem Ultraschallbild des Blutgefäßes mit der räumlichen Positionierung eines entsprechenden Blutgefäßes in Bezug auf entsprechende benachbarte anatomische Elemente in dem einen oder den mehreren Ultraschallbildern einschließt.

14. Verfahren nach einem der Ansprüche 9-13, weiter umfassend:
Umwandeln magnetischer Signale von einer magnetisierten medizinischen Vorrichtung mit einem Array von Magnetsensoren der Ultraschallsonde in entsprechende elektrische Signale der magnetischen Signale;
Verarbeiten der entsprechenden elektrischen Signale der magnetischen Signale mit dem Prozessor in Distanz- und Ausrichtungsinformationen in Bezug auf das Blutgefäß; und
Darstellen einer ikonografischen Darstellung der medizinischen Vorrichtung auf der Anzeige (104); und wobei das Verfahren wahlweise
1) weiter Folgendes umfasst:
Emittieren optischer Eingangssignale, Empfangen reflektierter optischer Signale und Umwandeln der reflektierten optischen Signale in entsprechende elektrische Signale der optischen Signale durch einen alleinstehenden optischen Interrogator (154), der kommunikativ mit der Konsole gekoppelt ist, oder einen integrierten optischen Interrogator, der in die Konsole integriert ist;
Leiten der optischen Eingangssignale von dem optischen Interrogator zu einer Anzahl von Faser-Bragg-Gitter(FBG)-Sensoren entlang einer Länge eines Lichtleitermandrins (156) und der reflektierten optischen Signale von der Anzahl von FBG-Sensoren zurück zu dem optischen Interrogator mit dem Lichtleitermandrin, der in einem Lumen der medizinischen Vorrichtung angeordnet ist;
Verarbeiten der entsprechenden elektrischen Signale der optischen Signale mit dem Prozessor in Distanz- und Ausrichtungsinformationen in Bezug auf das Blutgefäß; und
Darstellen einer ikonografischen Darstellung einer medizinischen Vorrichtung auf der Anzeige (104); und/oder
2) weiter das Anpassen der Distanz und Ausrichtung der aktivierten Ultraschallwandler in Bezug auf das Blutgefäß umfasst, wenn die medizinische Vorrichtung in die Nähe der Ultraschallsonde gebracht wird, wodurch eine Bildebene der Vorrichtung durch die aktivierten Ultraschallwandler senkrecht oder parallel zu einer Ebene der medizinischen Vorrichtung hergestellt wird, die die medizinische Vorrichtung zum Zugreifen auf das Blutgefäß mit der medizinischen Vorrichtung einschließt.

15. Verfahren nach einem der Ansprüche 9-14, weiter umfassend:
Bereitstellen von Positionsverfolgungsdaten für die Konsole von einem Beschleunigungsmesser (160), einem Gyroskop (162), einem Magnetometer (164) oder einer Kombination davon der Ultraschallsonde; und
Verarbeiten der Positionsverfolgungsdaten mit dem Prozessor für das Anpassen der Distanz der aktivierten Ultraschallwandler zu dem Blutgefäß, der Ausrichtung der aktivierten Ultraschallwandler zu dem Blutgefäß oder sowohl der Distanz als auch der Ausrichtung der aktivierten Ultraschallwandler in Bezug auf das Blutgefäß; und/oder weiter umfassend Aufrechterhalten der Distanz und der Ausrichtung der aktivierten Ultraschallwandler in Bezug auf das Blutgefäß, wenn die Ultraschallsonde unbeabsichtigt in Bezug auf das Blutgefäß bewegt wird; und/oder weiter umfassend das Darstellen des Ultraschallbildes des Blutgefäßes auf einer Anzeige (104), die mit der Konsole (102) gekoppelt ist.

## Revendications

1. Système (100) d'imagerie ultrasonore, comprenant :
une sonde ultrasonore (106) incluant un réseau de transducteurs ultrasonores (149), des transducteurs ultrasonores activés du réseau de transducteurs ultrasonores étant configurés pour émettre des signaux ultrasonores générés chez un patient, recevoir des signaux ultrasonores réfléchis provenant du patient et convertir les signaux ultrasonores réfléchis en signaux électriques correspondants des signaux ultrasonores en vue de leur traitement en images ultrasonores ;
une console (102) configurée pour communiquer avec la sonde ultrasonore, la console incluant un ou plusieurs processeurs et un support non transitoire lisible par ordinateur sur lequel une logique est stockée, laquelle,
lorsqu'elle est exécutée par les un ou plusieurs processeurs, provoque des opérations incluant :
la définition d'une image ultrasonore d'un vaisseau sanguin conformément à un plan d'image de la sonde ultrasonore ;
la détermination d'un désalignement entre le vaisseau sanguin et le plan d'image ; la fourniture d'une notification en réponse à la détermination du désalignement ; et le réglage d'une orientation des transducteurs ultrasonores activés pour orienter le plan d'image perpendiculairement au vaisseau sanguin.

2. Système d'imagerie ultrasonore selon la revendication 1, dans lequel l'image ultrasonore d'un vaisseau sanguin définit une forme elliptique ; et facultativement dans lequel la détermination du désalignement inclut :
l'identification d'une longueur et d'une largeur de la forme elliptique ;
le calcul d'un paramètre se rapportant à une différence entre la longueur et la largeur ; et la comparaison du paramètre calculé avec une limite de paramètre stockée en mémoire.

3. Système d'imagerie ultrasonore selon l'une quelconque des revendications précédentes, dans lequel la notification est tactile, audible, visuelle, ou toute combinaison de celles-ci.

4. Système d'imagerie ultrasonore selon l'une quelconque des revendications précédentes, dans lequel :
la sonde ultrasonore inclut une capacité d'ultrasons Doppler ; et
les opérations incluent en outre la détermination d'une direction de flux sanguin à l'intérieur du vaisseau sanguin par rapport au plan d'image sur la base de données d'ultrasons Doppler ; et facultativement dans lequel le rendu de l'image ultrasonore du vaisseau sanguin inclut la superposition d'un repère par-dessus l'image ultrasonore du vaisseau sanguin, le repère indiquant la direction du flux sanguin.

5. Système d'imagerie ultrasonore selon l'une quelconque des revendications précédentes, dans lequel les opérations incluent en outre :
la comparaison de l'image ultrasonore du vaisseau sanguin avec une ou plusieurs images ultrasonores de vaisseaux sanguins stockées en mémoire, les une ou plusieurs images ultrasonores se rapportant à une procédure médicale définie ; et
la fourniture d'une notification lorsque, à la suite de la comparaison, une orientation du plan d'image de l'image ultrasonore du vaisseau sanguin est déterminée comme étant opposée à une orientation d'un plan d'image correspondant des une ou plusieurs images ultrasonores ; et
facultativement dans lequel la comparaison de l'image ultrasonore inclut la comparaison du positionnement spatial du vaisseau sanguin par rapport à des éléments anatomiques adjacents dans l'image ultrasonore du vaisseau sanguin avec le positionnement spatial d'un vaisseau sanguin correspondant par rapport à des éléments anatomiques adjacents correspondants dans les une ou plusieurs images ultrasonores.

6. Système d'imagerie ultrasonore selon l'une quelconque des revendications précédentes, la sonde ultrasonore comprenant en outre : un réseau (146) de capteurs magnétiques (150) configuré pour convertir des signaux magnétiques provenant d'un dispositif médical magnétisé en signaux électriques correspondants des signaux magnétiques pour traitement par le processeur en informations de distance et d'orientation par rapport au vaisseau sanguin en vue de l'affichage d'une représentation iconographique du dispositif médical sur l'écran d'affichage ; et facultativement dans lequel la distance et l'orientation des transducteurs ultrasonores activés sont réglées par rapport au vaisseau sanguin lorsque le dispositif médical est amené à proximité de la sonde ultrasonore, un plan d'image de dispositif établi par les transducteurs ultrasonores activés étant perpendiculaire ou parallèle à un plan de dispositif médical incluant le dispositif médical pour accéder au vaisseau sanguin avec le dispositif médical.

7. Système d'imagerie ultrasonore selon la revendication 6, comprenant en outre :
un interrogateur optique autonome (154) couplé en communication à la console ou un interrogateur optique intégré qui est intégré dans la console, l'interrogateur optique étant configuré pour émettre des signaux optiques d'entrée, recevoir des signaux optiques réfléchis et convertir les signaux optiques réfléchis en signaux électriques correspondants des signaux optiques pour traitement par le processeur en informations de distance et d'orientation par rapport au vaisseau sanguin en vue de l'affichage d'une représentation iconographique du dispositif médical sur l'écran ; et
un stylet (156) à fibre optique configuré pour acheminer les signaux optiques d'entrée depuis l'interrogateur optique vers un certain nombre de capteurs à réseau de Bragg sur fibre, FBG, le long d'une longueur du stylet à fibre optique et les signaux optiques réfléchis depuis le certain nombre de capteurs FBG de retour vers l'interrogateur optique, le stylet à fibre optique étant configuré pour être disposé dans une lumière du dispositif médical.

8. Système d'imagerie ultrasonore selon l'une quelconque des revendications précédentes, la sonde ultrasonore comprenant en outre un accéléromètre (160), un gyroscope (162), un magnétomètre (164), ou une combinaison de ceux-ci, configurés pour fournir des données de suivi de position à la console, le processeur étant en outre configuré pour exécuter les instructions pour le traitement des données de suivi de position pour le réglage de la distance des transducteurs ultrasonores activés par rapport au vaisseau sanguin, de l'orientation des transducteurs ultrasonores activés par rapport au vaisseau sanguin, ou à la fois de la distance et de l'orientation des transducteurs ultrasonores activés par rapport au vaisseau sanguin ; et/ou, dans lequel la distance et l'orientation des transducteurs ultrasonores activés sont maintenues par rapport au vaisseau sanguin lorsque la sonde ultrasonore est déplacée par inadvertance par rapport au vaisseau sanguin ; et/ou dans lequel les opérations incluent en outre le rendu de l'image ultrasonore du vaisseau sanguin sur un dispositif d'affichage (104) du système.

9. Procédé d'un système d'imagerie ultrasonore (100) incluant un support lisible par ordinateur, CRM, non transitoire présentant des instructions exécutables qui amènent le système d'imagerie ultrasonore à mettre en œuvre un ensemble d'opérations pour l'imagerie ultrasonore lorsque les instructions sont exécutées par un processeur (116) d'une console (102) du système d'imagerie ultrasonore, le procédé comprenant :
l'activation de transducteurs ultrasonores (149) d'un réseau (148) de transducteurs ultrasonores d'une sonde ultrasonore couplée en communication à la console, de telle sorte que les transducteurs ultrasonores émettent des signaux ultrasonores générés chez un patient, reçoivent des signaux ultrasonores réfléchis provenant du patient et convertissent les signaux ultrasonores réfléchis en signaux électriques correspondants des signaux ultrasonores pour traitement en images ultrasonores ;
la définition d'une image ultrasonore d'un vaisseau sanguin conformément à un plan d'image de la sonde ultrasonore,
la détermination d'un désalignement entre le vaisseau sanguin et le plan d'image ;
la fourniture d'une notification en réponse à la détermination du désalignement ; et
le réglage d'une orientation des transducteurs ultrasonores activés pour orienter le plan d'image perpendiculairement au vaisseau sanguin.

10. Procédé selon la revendication 9, dans lequel l'image ultrasonore d'un vaisseau sanguin définit une forme elliptique, et facultativement dans lequel la détermination du désalignement comprend :
l'identification d'une longueur et d'une largeur de la forme elliptique ;
le calcul d'un paramètre se rapportant à une différence entre la longueur et la largeur ; et
la comparaison du paramètre calculé avec une limite de paramètre stockée en mémoire.

11. Procédé selon l'une quelconque des revendications 9 ou revendication 10, dans lequel la notification est tactile, audible, visuelle, ou toute combinaison de celles-ci.

12. Procédé selon l'une quelconque des revendications 9 à 11, dans lequel la sonde ultrasonore inclut une capacité d'ultrasons Doppler, le procédé comprenant en outre la détermination d'une direction de flux sanguin à l'intérieur du vaisseau sanguin par rapport au plan d'image sur la base de données d'ultrasons Doppler ; et facultativement dans lequel le rendu de l'image ultrasonore du vaisseau sanguin inclut la superposition d'un repère au-dessus de l'image ultrasonore du vaisseau sanguin, le repère indiquant la direction du flux sanguin.

13. Procédé selon l'une quelconque des revendications 9 à 12, comprenant en outre :
la comparaison de l'image ultrasonore du vaisseau sanguin avec une ou plusieurs images ultrasonores de vaisseaux sanguins stockées en mémoire, les une ou plusieurs images ultrasonores se rapportant à une procédure médicale définie ; et
la fourniture d'une notification lorsque, à la suite de la comparaison,
une orientation du plan d'image de l'image ultrasonore du vaisseau sanguin est déterminée comme étant opposée à l'orientation du plan d'image des une ou plusieurs images ultrasonores ; et facultativement dans lequel la comparaison de l'image ultrasonore inclut la comparaison du positionnement spatial du vaisseau sanguin par rapport à des éléments anatomiques adjacents dans l'image ultrasonore du vaisseau sanguin avec le positionnement spatial d'un vaisseau sanguin correspondant par rapport à des éléments anatomiques adjacents correspondants dans les une ou plusieurs images ultrasonores.

14. Procédé selon l'une quelconque des revendications 9 à 13, comprenant en outre :
la conversion de signaux magnétiques provenant d'un dispositif médical magnétisé avec un réseau de capteurs magnétiques de la sonde ultrasonore en signaux électriques correspondants des signaux magnétiques ;
le traitement des signaux électriques correspondants des signaux magnétiques avec le processeur en informations de distance et d'orientation par rapport au vaisseau sanguin ; et
le rendu d'une représentation iconographique du dispositif médical sur le dispositif d'affichage (104) ; et le procédé facultativement
1) comprenant en outre :
l'émission de signaux optiques d'entrée, la réception de signaux optiques réfléchis et la conversion des signaux optiques réfléchis en signaux électriques correspondants des signaux optiques par un interrogateur optique autonome (154) couplé en communication à la console ou un interrogateur optique intégré qui est intégré dans la console ;
l'acheminement des signaux optiques d'entrée depuis l'interrogateur optique vers un certain nombre de capteurs à réseau de Bragg sur fibre, FBG, le long d'une longueur d'un stylet (156) à fibre optique et des signaux optiques réfléchis depuis le certain nombre de capteurs FBG de retour vers l'interrogateur optique avec le stylet à fibre optique disposé dans une lumière du dispositif médical ;
le traitement des signaux électriques correspondants des signaux optiques avec le processeur en informations de distance et d'orientation par rapport au vaisseau sanguin ; et
le rendu d'une représentation iconographique d'un dispositif médical sur le dispositif d'affichage (104) ;
et/ou
2) comprenant en outre le réglage de la distance et de l'orientation des transducteurs ultrasonores activés par rapport au vaisseau sanguin lorsque le dispositif médical est amené à proximité de la sonde ultrasonore, établissant ainsi un plan d'image de dispositif par les transducteurs ultrasonores activés perpendiculaire ou parallèle à un plan de dispositif médical incluant le dispositif médical pour accéder au vaisseau sanguin avec le dispositif médical.

15. Procédé selon l'une quelconque des revendications 9 à 14, comprenant en outre :
la fourniture, à la console, de données de suivi de position depuis un accéléromètre (160), un gyroscope (162), un magnétomètre (164), ou une combinaison de ceux-ci de la sonde ultrasonore ; et
le traitement des données de suivi de position, à l'aide du processeur, pour le réglage de la distance des transducteurs ultrasonores activés par rapport au vaisseau sanguin, de l'orientation des transducteurs ultrasonores activés par rapport au vaisseau sanguin, ou à la fois de la distance et de l'orientation des transducteurs ultrasonores activés par rapport au vaisseau sanguin ; et/ou comprenant en outre le maintien de la distance et de l'orientation des transducteurs ultrasonores activés par rapport au vaisseau sanguin lorsque la sonde ultrasonore est déplacée par inadvertance par rapport au vaisseau sanguin ; et/ou comprenant en outre le rendu de l'image ultrasonore du vaisseau sanguin sur un dispositif d'affichage (104) couplé à la console (102).
